(19) Europäisches Patentamt
European Patent Office
Office européen des brevets

(11) Veröffentlichungsnummer: **0 323 408 B1**

(12) **EUROPÄISCHE PATENTSCHRIFT**

(45) Veröffentlichungstag der Patentschrift: **06.04.94**

(51) Int. Cl.[5]: **C07D 249/20**, G03C 1/46, C09B 63/00

(21) Anmeldenummer: **88810887.5**

(22) Anmeldetag: **21.12.88**

Die Akte enthält technische Angaben, die nach dem Eingang der Anmeldung eingereicht wurden und die nicht in dieser Patentschrift enthalten sind.

# (54) Neue 2-(2-Hydroxyphenyl)-benztriazol-derivate.

(30) Priorität: **28.12.87 CH 5075/87**

(43) Veröffentlichungstag der Anmeldung:
**05.07.89 Patentblatt 89/27**

(45) Bekanntmachung des Hinweises auf die Patenterteilung:
**06.04.94 Patentblatt 94/14**

(84) Benannte Vertragsstaaten:
**BE DE FR GB IT NL SE**

(56) Entgegenhaltungen:
**EP-A- 0 057 160**
**EP-A- 0 131 468**
**EP-A- 0 133 164**
**EP-A- 0 191 582**
**DE-A- 3 624 811**
**GB-A- 1 169 859**
**JP-B-50 159 484**
**US-A- 3 399 173**

**CHEMICAL ABSTRACTS, Band 107, Nr. 4, 27 Juli 1987, Columbus, Ohio, USA; NAKAMURA, SHINICHI; ISHIKAWA, HISASHI (KONISHIROKU PHOTO INDUSTRY CO., LTD.) "Silver halide color photographic photosensitive materials" Seite 519, Spalte 1, Zusamenfassung-Nr. 31129f**

(73) Patentinhaber: **CIBA-GEIGY AG**
**Klybeckstrasse 141**
**CH-4002 Basel(CH)**

(72) Erfinder: **Leppard, David G., Dr.**
**Route de Bourguillon 6**
**CH-1723 Marly(CH)**
Erfinder: **Slongo, Mario, Dr.**
**Sägetrainweg 553**
**CH-1712 Tafers(CH)**
Erfinder: **Rody, Jean, Dr.**
**Rütiring 82**
**CH-4125 Riehen(CH)**

**EP 0 323 408 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft neue Derivate des 2-(2-Hydroxyphenyl)-benztriazols und ihre Verwendung als Stabilisatoren.

Es sind verschiedene Derivate des 2-(2-Hydroxyphenyl)-benztriazols bekannt, die UV-Absorber sind und als Lichtschutzmittel für verschiedene Substrate, insbesondere für organische Polymere, verwendet werden. Die meisten dieser Derivate sind kristalline Verbindungen von relativ hohem Schmelzpunkt. Für bestimmte Substrate wie z.B. für Lacke oder für photographische Bildaufzeichnungsmaterialien sucht man nach Lichtschutzmitteln mit UV-absorbierenden Eigenschaften, welche niedrig-schmelzende oder flüssige Verbindungen sind. Sie sollen ausserdem schwer-flüchtig sein.

In der EP-A-57 160 werden Derivate der 3-(Benztriazolyl-2)-4-hydroxy-5-tert.butylphenylpropionsäure beschrieben, die niedrig-schmelzend sind und sich in hochsiedenden Lösungsmitteln gut lösen. Für eine Verwendung ohne Lösungsmittel ist ihre Viskosität jedoch zu hoch.

In der EP-A-189 374 wird das Problem dadurch gelöst, dass man technische Gemische von isomeren Alkylierungsprodukten verwendet. Dies hat den Nachteil, dass es schwierig ist, technische Produkte gleichbleibender Zusammensetzung reproduzierbar herzustellen. Ausserdem ist es schwierig, in solchen Gemischen Verunreinigungen analytisch festzustellen.

Aus JP-B2-50-159 484 sind Hydroxyphenylbenztriazole bekannt, die durch Carboxylalkylsubstituenten am Hydroxyphenylring gekennzeichnet sind und sich insbesondere zur Verwendung in Kosmetika eignen.

Es wurden jetzt neue Benztriazol-Derivate entwickelt, die in hervorragender Weise als Lichtschutzmittel verwendbar sind.

Es handelt sich hierbei um Verbindungen der Formel I

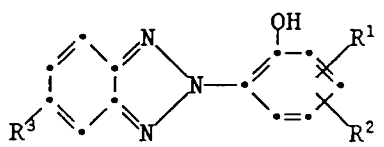

I

worin $R^1$ in ortho- oder para-Stellung zur OH-Gruppe steht und eine Gruppe der Formel II bedeutet,

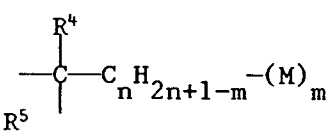

II

worin $R^4$ und $R^5$ gleich oder verschieden sind und $C_1$-$C_5$-Alkyl bedeuten oder $R^4$ zusammen mit dem Rest $C_nH_{2n+1-m}$ einen $C_5$-$C_{12}$-Cycloalkylenring bildet,
m 1 oder 2 bedeutet,
n eine ganze Zahl von 2 bis 20 ist und
M einer der folgenden Reste ist:
a) -$COOR^6$ oder

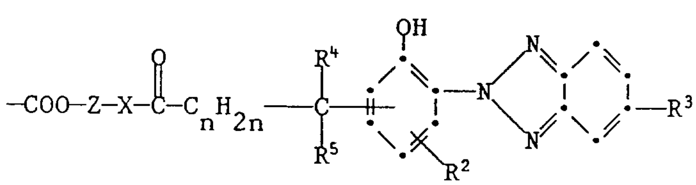

worin $R^6$ Wasserstoff, unsubstituiertes oder durch -OH, -O-CO-$R^7$ oder -P(O)($OR^8$)$_2$ substituiertes $C_1$-$C_{18}$-Alkyl, durch ein oder mehrere -O-, -S-oder -N($R^9$)- unterbrochenes $C_3$-$C_{30}$-Alkyl oder -Hydroxyalkyl, unsubstituiertes oder durch -OH substituiertes $C_5$-$C_{12}$-Cycloalkyl, $C_2$-$C_{18}$-Alkenyl, $C_7$-$C_{15}$-Aralkyl, Glycidyl, Furfuryl, eine Gruppe -$CH_2$-CH(OH)-$R^{10}$, einen einwertigen Mono- oder Disaccharid-Rest oder eine Gruppe der Formel III

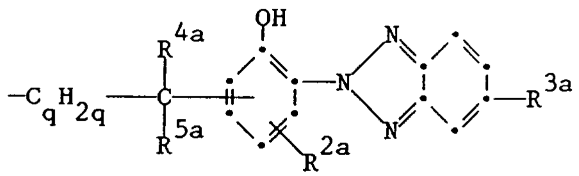

(III)

bedeutet, worin q eine ganze Zahl von 2 bis 20 ist und $R^{4a}$ und $R^{5a}$ $C_1$-$C_5$-Alkyl bedeuten,
X -O- oder -N($R^9$)- bedeutet,
Z $C_2$-$C_8$-Alkylen, $C_4$-$C_8$-Alkenylen, $C_4$-$C_8$-Alkinylen, Cyclohexylen, durch ein oder mehrere -O- unterbrochenes $C_4$-$C_{40}$-Alkylen oder $-CH_2CH(OH)CH_2-OR^{11}O-CH_2CH(OH)CH_2-$ bedeutet,
$R^7$ $C_1$-$C_{18}$-Alkyl, $C_5$-$C_{12}$-Cycloalkyl, $C_2$-$C_8$-Alkenyl, $C_6$-$C_{10}$-Aryl, $C_7$-$C_{18}$-Alkylaryl oder $C_7$-$C_{15}$-Aralkyl bedeutet,
$R^8$ $C_1$-$C_{18}$-Alkyl, $C_5$-$C_{12}$-Cycloalkyl, $C_3$-$C_8$-Alkenyl, $C_6$-$C_{10}$-Aryl oder $C_7$-$C_{15}$-Aralkyl bedeutet,
$R^9$ Wasserstoff, $C_1$-$C_{18}$-Alkyl, $C_5$-$C_{12}$-Cycloalkyl, $C_3$-$C_8$-Alkenyl, $C_6$-$C_{10}$-Aryl, $C_7$-$C_{18}$-Alkylaryl oder $C_7$-$C_{15}$-Aralkyl bedeutet,
$R^{10}$ Wasserstoff, $C_1$-$C_{12}$-Alkyl, Phenyl, Hydroxyphenyl, $C_7$-$C_{15}$-Aralkyl oder $-CH_2OR^7$ bedeutet und
$R^{11}$ $C_2$-$C_8$-Alkylen, durch -O- unterbrochenes $C_4$-$C_{10}$-Alkylen, Cyclohexylen, Phenylen oder eine Gruppe

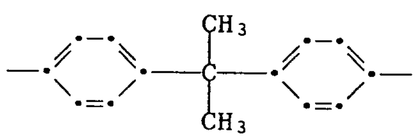

oder

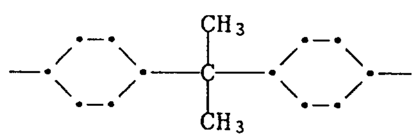

bedeutet,
b) -CO-N($R^{12}$)($R^{13}$) oder

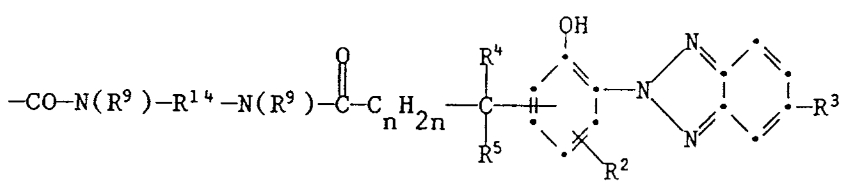

,

worin $R^{12}$ und $R^{13}$ unabhängig voneinander H, $C_1$-$C_{18}$-Alkyl, durch ein oder mehrere -O-, -S- oder -N($R^9$)- unterbrochenes $C_3$-$C_{30}$-Alkyl, $C_5$-$C_{12}$-Cycloalkyl, Phenyl oder durch $C_1$-$C_{12}$-Alkyl, $C_1$-$C_4$-Alkoxy oder Halogen substituiertes Phenyl, $C_3$-$C_6$-Alkenyl, $C_7$-$C_{15}$-Aralkyl, $C_2$-$C_4$-Hydroxyalkyl oder eine Gruppe der Formel III bedeuten oder
$R^{12}$ und $R^{13}$ zusammen $C_4$-$C_6$-Alkylen oder durch -O-, -S- oder -N($R^9$)-unterbrochenes $C_4$-$C_6$-Alkylen bedeuten und
$R^{14}$ $C_2$-$C_{12}$-Alkylen, durch -O- unterbrochenes $C_4$-$C_{12}$-Alkylen, Cyclohexylen, Phenylen oder eine Gruppe

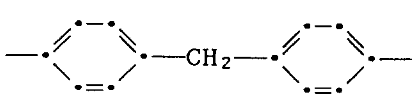

oder

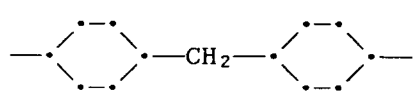

bedeutet oder zusammen mit den beiden N-Atomen und den beiden Resten $R^9$ einen Piperazinring bildet,

c) $-OR^{12}$ oder

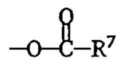

wobei $R_7$ verschieden von Vinyl und Isopropenyl ist, wenn $R^2$ Wasserstoff, Methyl oder tert. Alkyl mit 4 bis 6 Kohlenstoffatomen ist, und $R^{12}$ verschieden von Wasserstoff ist, wenn $R^2$ in ortho-Stellung zur OH-Gruppe steht und Wasserstoff, Methyl oder Alkyl mit 4 bis 6 Kohlenstoffatomen ist.

d) $-N(R^{16})(R^{17})$,
worin $R^{16}$ Wasserstoff oder $C_1$-$C_{12}$-Alkyl ist und
$R^{17}$ Wasserstoff, $C_1$-$C_{12}$-Alkyl oder $-CO-R^9$ ist oder
$R^{16}$ und $R^{17}$ zusammen $C_4$-$C_6$-Alkylen oder durch -O-, -S- oder $-N(R^9)$-unterbrochenes $C_4$-$C_6$-Alkylen bedeuten,

e) $-P(O)(OR^{18})(OR^{19})$ oder $-P(O)(OR^{18})-R^{20}$,
worin $R^{18}$ Wasserstoff oder $C_1$-$C_{18}$-Alkyl ist,
$R^{19}$ $C_1$-$C_{18}$-Alkyl ist, oder $R^{18}$ und $R^{19}$ zusammen $C_2$-$C_5$-Alkylen bedeuten und
$R^{20}$ $C_1$-$C_{12}$-Alkyl oder Phenyl bedeutet,

f) -CN, $-NO_2$ oder Halogen,

g)

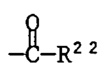 ,

worin $R^{22}$ Wasserstoff, $C_1$-$C_{20}$-Alkyl oder Halogen bedeutet;

$R^2$ und $R^{2a}$ in ortho- oder para-Stellung zur OH-Gruppe stehen und Wasserstoff, Halogen, $C_1$-$C_{18}$-Alkyl, $C_7$-$C_9$-Phenylalkyl, $C_5$-$C_8$-Cycloalkyl, Phenyl, eine Gruppe der Formel II oder eine Gruppe der Formel IV, V oder VI bedeuten

$-C_pH_{2p}-COOR^6$ IV

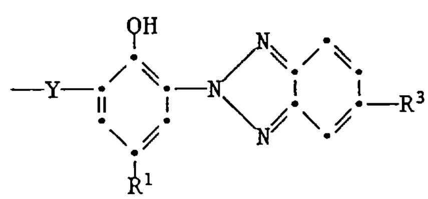

V

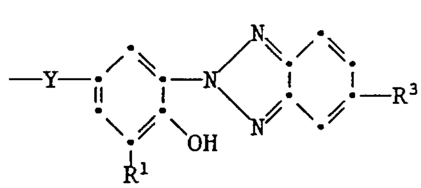

VI

worin p 0,1 oder 2 ist und Y eine direkte Bindung, -S- oder eine Gruppe

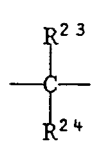

ist, worin $R^{23}$ und $R^{24}$ unabhängig voneinander Wasserstoff, $C_1$-$C_{18}$-Alkyl oder durch 1 bis 3 -S-unterbrochenes $C_3$-$C_{20}$-Alkyl bedeuten und

$R^3$ und $R^{3a}$ Wasserstoff, Chlor, $C_1$-$C_4$-Alkyl, $C_1$-$C_4$-Alkoxy oder -$COOR^6$ bedeuten.

Soweit darin irgendwelche Substituenten Alkyl sind, können sie geradkettiges oder verzweigtes Alkyl sein. Dasselbe gilt für substituierte oder durch Heteroatome unterbrochene Alkylgruppen. Soweit irgendwelche Substituenten Alkylen oder durch Heteroatome unterbrochenes Alkylen sind, kann es sich um unverzweigte oder verzweigte Ketten handeln.

$R^6$ als durch O unterbrochenes Hydroxyalkyl ist vorzugsweise eine Gruppe

$$-(CH_2CH_2O)_x-H,$$

worin x 1-12 ist.

$R^7$, $R^8$ und $R^9$ als Alkylaryl sind vorzugsweise Alkylphenyl. Wenn $R^4$ zusammen mit dem Rest $C_nH_{2n+1-m}$ einen $C_5$-$C_{12}$-Cycloalkylenring bildet, so handelt es sich vorzugsweise um einen Cyclohexanring. Wenn m 2 ist, so können die beiden Gruppen M an demselben C-Atom oder an zwei verschiedenen C-Atomen der Gruppe $C_nH_{2n-1}$ sitzen. Wenn m 1 ist, so ist M bevorzugt an das Ende der Gruppe $C_nH_{2n}$ gebunden.

Wenn $R^{12}$ und $R^{13}$ oder $R^{16}$ und $R^{17}$ Alkylen oder durch O, S oder $N(R^9)$ unterbrochenes Alkylen bedeuten, so bilden sie zusammen mit dem N-Atom, an das sie gebunden sind, einen gesättigten heterocyclischen Ring, beispielsweise einen Pyrrolidin-, Piperidin-, Morpholin-, Thiomorpholin-, Piperazin- oder 4-Methylpiperazin-Ring.

Der Substituent M ist bevorzugt

a) eine Gruppe -$COOR^6$ oder

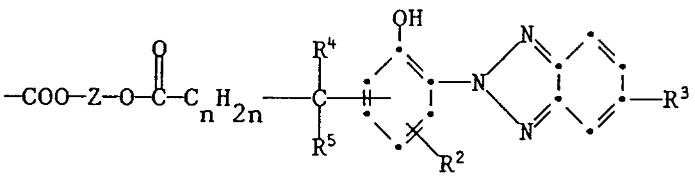

,

worin $R^6$ Wasserstoff, unsubstituiertes oder durch -OH substituiertes $C_1$-$C_{18}$-Alkyl, durch ein oder mehrere -O- unterbrochenes $C_3$-$C_{20}$-Alkyl oder -Hydroxyalkyl, unsubstituiertes oder durch -OH substituiertes $C_5$-$C_{12}$-Cycloalkyl, $C_2$-$C_{18}$-Alkenyl, $C_7$-$C_{15}$-Aralkyl, Glycidyl, Furfuryl oder eine Gruppe -$CH_2$-CH(OH)-$R^{10}$ bedeutet und $R^{10}$ Wasserstoff, $C_1$-$C_{12}$-Alkyl, $C_7$-$C_{12}$-Aralkyl oder -$CH_2OR^7$ bedeutet und $R^7$ $C_1$-$C_{18}$-Alkyl, Cyclohexyl, Phenyl, Tolyl oder Benzyl ist,

Z $C_2$-$C_8$-Alkylen, $C_4$-$C_8$-Alkenylen, Cyclohexylen, durch ein oder mehrere -O- unterbrochenes $C_4$-$C_{40}$-Alkylen oder -$CH_2CH(OH)CH_2$-$OR^{11}$-O-$CH_2CH(OH)CH_2$-bedeutet,

$R^{11}$ $C_2$-$C_8$-Alkylen oder durch ein oder mehrere -O- unterbrochenes $C_4$-$C_{10}$-Alkylen bedeutet und $R^2$, $R^3$, $R^4$, $R^5$ und n die vorhin gegebenen Bedeutungen haben;

b) eine Gruppe -CO-$N(R^{12})(R^{13})$ oder

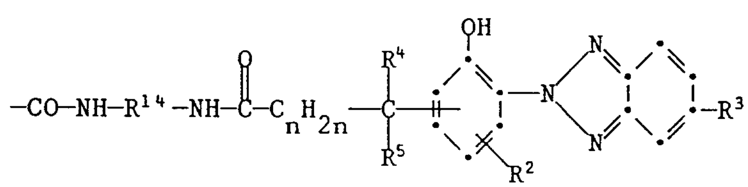

,

worin $R^{12}$ und $R^{13}$ unabhängig voneinander H, $C_1$-$C_{12}$-Alkyl, durch -O-unterbrochenes $C_3$-$C_{20}$-Alkyl, $C_5$-$C_{12}$-Cycloalkyl, Phenyl oder durch $C_1$-$C_{12}$-Alkyl, $C_1$-$C_4$-Alkoxy oder Halogen substituiertes Phenyl, $C_3$-$C_8$-Alkenyl, $C_7$-$C_{11}$-Phenylalkyl, oder $C_2$-$C_4$-Hydroxyalkyl bedeuten oder $R^{12}$ und $R^{13}$ zusammen $C_4$-$C_5$-Alkylen oder durch -O- oder -$N(R^9)$-unterbrochenes $C_4$-$C_6$-Alkylen bedeuten,

$R^{14}$ $C_2$-$C_8$-Alkylen oder durch -O- unterbrochenes $C_4$-$C_8$-Alkylen bedeutet $R^1$, $R^2$, $R^3$, $R^4$, $R^5$ und n die vorhin gegebenen Bedeutungen haben;

c) eine Gruppe $-OR^{12}$ oder

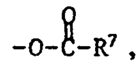

worin $R^{12}$ Wasserstoff, $C_1$-$C_{12}$-Alkyl, $C_5$-$C_{12}$-Cycloalkyl, $C_3$-$C_6$-Alkenyl, Phenyl oder $C_7$-$C_{11}$-Phenylalkyl bedeutet und $R_7$ $C_1$-$C_{18}$-Alkyl, Cyclohexyl, $C_2$-$C_6$-Alkenyl, Phenyl oder $C_7$-$C_{12}$-Alkylphenyl bedeutet;
d) eine Gruppe $-P(O)(OR^{18})(OR^{19})$ oder $-P(O)(OR^{18})-R^{20}$ ist, worin $R^{18}$ Wasserstoff oder $C_1$-$C_{12}$-Alkyl, $R^{19}$ $C_1$-$C_{12}$-Alkyl ist, und $R^{20}$ $C_1$-$C_{12}$-Alkyl oder Phenyl bedeutet; oder
e) eine Gruppe

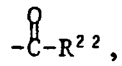

worin $R^{22}$ $C_1$-$C_{12}$-Alkyl bedeutet.

Besonders bevorzugt ist M eine Gruppe $-COOR^6$.

Bevorzugt sind Verbindungen der Formel I, worin $R^1$ eine Gruppe der Formel IIa ist,

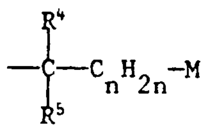
IIa

worin $R^4$ und $R^5$ unabhängig voneinander $C_1$-$C_5$-Alkyl sind oder $R^4$ zusammen mit dem Rest $C_nH_{2n}$ einen $C_5$-$C_6$-Cycloalkylenring bildet,
n eine ganze Zahl von 2 bis 8 ist,
M eine der vorhin angegebenen Bedeutungen hat,
$R^2$ $C_1$-$C_{12}$-Alkyl oder eine Gruppe der Formel IIa oder eine Gruppe der Formel IV bedeutet, worin p 1 oder 2 ist, und
$R^3$ Wasserstoff, Chlor, $C_1$-$C_4$-Alkyl, $C_1$-$C_4$-Alkoxy oder $-COOR^6$ bedeutet, worin $R^6$ eine der vorhin angegebenen Bedeutungen hat, insbesondere solche Verbindungen der Formel I, worin $R^1$ eine Gruppe der Formel IIa ist, worin $R^4$ und $R^5$ Methyl sind und n eine ganze Zahl von 2 bis 6 ist,
M eine Gruppe $-COOR^6$ oder -OH ist, worin $R^6$ Wasserstoff, unsubstituiertes oder durch -OH substituiertes $C_1$-$C_{18}$-Alkyl, durch ein oder mehrere -O- unterbrochenes $C_3$-$C_{30}$-Alkyl oder -Hydroxyalkyl, unsubstituiertes oder durch -OH substituiertes $C_5$-$C_{12}$-Cycloalkyl, $C_2$-$C_{18}$-Alkenyl, $C_7$-$C_{15}$-Aralkyl, Glycidyl oder Furfuryl bedeutet,
$R^2$ $C_1$-$C_{12}$-Alkyl, $C_7$-$C_9$-Phenylalkyl, $C_5$-$C_8$-Cycloalkyl, eine Gruppe der Formel II oder der Formel IV bedeutet, worin p 1 oder 2 ist und
$R^3$ Wasserstoff, Chlor, $C_1$-$C_4$-Alkyl, $C_1$-$C_4$-Alkoxy oder $-COOR^6$ bedeutet.

Besonders bevorzugt sind Verbindungen der Formel I, worin $R^1$ eine Gruppe der Formel IIb ist,

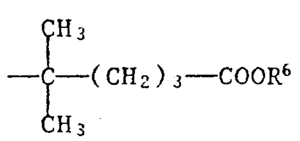
IIb

worin $R^6$ unsubstituiertes oder durch -OH substituiertes $C_5$-$C_{18}$-Alkyl, durch ein oder mehrere -O- unterbrochenes $C_3$-$C_{30}$-Alkyl oder -Hydroxyalkyl, $C_5$-$C_{12}$-Cycloalkyl, $C_2$-$C_{18}$-Alkenyl, $C_7$-$C_{15}$-Aralkyl, Glycidyl oder Furfuryl bedeutet,
$R^2$ $C_1$-$C_{12}$-Alkyl, Cyclohexyl oder eine Gruppe der Formel IIb oder IV bedeutet, worin p 1 oder 2 ist, und
$R^3$ Wasserstoff, Chlor, $C_1$-$C_4$-Alkyl oder $C_1$-$C_4$-Alkoxy bedeutet.

Besonders bevorzugt sind Verbindungen der Formel Ia,

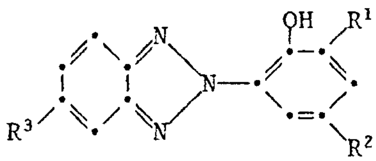

Ia

worin $R^1$ eine Gruppe der Formel IIb ist,
worin $R^6$ $C_5$-$C_{12}$-Alkyl, durch ein oder mehrere -O-unterbrochenes $C_3$-$C_{18}$-Alkyl, $C_5$-$C_{12}$-Cycloalkyl, $C_3$-$C_{18}$-Alkenyl oder $C_7$-$C_{12}$-Phenylalkyl bedeutet,
$R^2$ dieselbe Bedeutung wie $R^1$ hat oder $C_1$-$C_{12}$-Alkyl oder eine Gruppe der Formel IV ist, worin p 1 oder 2 bedeutet, und
$R^3$ Wasserstoff, Chlor oder $C_1$-$C_4$-Alkyl bedeutet.

Beispiele für Verbindungen der Formel I sind die folgenden Verbindungen der Formel

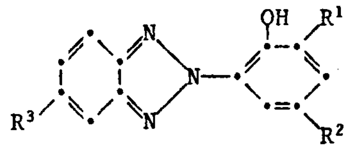

| $R^1$ | $R^2$ | $R^3$ |
|---|---|---|
| $-C(CH_3)_2CH_2CH_2CH_2COOCH_3$ | $-CH_3$ | H |
| $-C(CH_3)_2CH_2CH_2CH_2COOC_8H_{17}$ | $-C_2H_5$ | H |
| $-C(CH_3)_2CH_2CH_2CH_2COOC_8H_{17}$ | $-C(CH_3)_2CH_2CH_2CH_2COOC_8H_{17}$ | H |
| $-C(CH_3)_2CH_2CH_2CH_2COOC_6H_{13}$ | $-CH(CH_3)_2$ | H |
| $-C(CH_3)_2CH_2CH_2CH_2COOC_{12}H_{25}$ | $-C(CH_3)_3$ | H |
| $-C(CH_3)_2CH_2CH_2CH_2COOC_4H_9$ | $-C(CH_3)_2CH_2C(CH_3)_3$ | H |
| $-C(CH_3)_2CH_2CH_2CH_2COO(CH_2CH_2O)_3H$ | $-C(CH_3)_2C_2H_5$ | H |
| $-C(CH_3)_2CH_2CH_2P(O)(OCH_3)(OH)$ | $-CH_3$ | H |
| $-C(CH_3)_2CH_2CH_2CH_2CN$ | $-CH_2$Phenyl | H |
| $-C(CH_3)_2CH_2CH_2CH_2CH(CH_3)NHCOCH_3$ | $-CH(CH_3)C_2H_5$ | H |
| $-C(CH_3)_2CH_2CH_2CH_2COOCH_2CH(C_2H_5)C_4H_9$ | $-CH_3$ | $-COOCH_2CH(C_2H_5)C_4H_9$ |
| $-C(CH_3)_2-(CH_2)_3-CH(CH_3)-CH_2CH_2OH$ | $-C_2H_5$ | H |
| $-C(CH_3)_2CH_2CH_2CH_2COOC_8H_{17}$ | $-CH_3$ | Cl |
| $-C(CH_3)_2CH_2CH_2CH_2COOC_8H_{17}$ | $-CH(CH_3)_2$ | $-C_2H_5$ |
| $-C(CH_3)_2CH_2CH_2CH_2COOC_8H_{17}$ | $-CH_2COOC_8H_{17}$ | H |
| $-C(CH_3)_2CH_2CH_2CH_2COOC_2H_5$ | $-CH_2CH_2COOC_2H_5$ | H |
| $-C(CH_3)_2CH_2CH_2CH_2COOC_8H_{17}$ | $-CH_2CH_2COOC_8H_{17}$ | H |
| $-C(CH_3)_2CH_2CH_2CH_2COOC_8H_{17}$ | $-C(CH_3)_2$-Phenyl | H |
| $-C(CH_3)_2CH_2CH_2CH(COOC_4H_9)_2$ | $-CH_3$ | H |
| $-C(CH_3)_2-(CH_2)_3-CH(CH_3)-CH_2CH_2OCOCH_3$ | $-C(CH_3)_3$ | H |
| $-C(CH_3)_2CH_2CH_2CH_2COCH_3$ | -Phenyl | H |
| $-C(CH_3)_2CH_2CH_2CH_2NHC_8H_{17}$ | $-C(CH_3)_2CH_2CH_2CH_2NHC_8H_{17}$ | H |
| $-C(CH_3)_2CH_2CH_2CH_2COOC_8H_{17}$ | -Cl | H |
| $-C(CH_3)_2CH_2CH_2CH_2COOC_8H_{17}$ | -Cyclohexyl | H |
| $-C(CH_3)_2CH_2CH_2CH_2CH(CH_3)CH_2CH_2Br$ | $-CH(CH_3)C_2H_5$ | H |
| 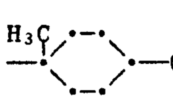 $COOCH_2CH(CH_3)C_2H_5$ | $-CH_3$ | H |
| 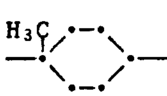 $CONHCH_2CH(C_2H_5)C_4H_9$ | $-CH_3$ | H |

| | | |
|---|---|---|
| $-C_2H_5$ | $-C(CH_3)_2CH_2CH2CH_2COOC_5H_{11}$ | H |
| $-CH(CH_3)_2$ | $-C(CH_3)_2CH_2CH_2CH_2COOCyclohexyl$ | H |
| $-CH_3$ | $-C(CH_3)_2CH_2CH_2CH_2COCH_3$ | H |
| $-CH(CH_3)C_2H_5$ | $-C(CH_3)_2CH_2CH_2CH_2CN$ | H |
| $-CH(CH_3)_2$ | $-C(CH_3)_2CH_2CH_2CH_2COOCH_2CH(CH_3)C_2H_5$ | Cl |
| -H | $-C(CH_3)_2CH_2CH_2CH_2CH(CH_3)NHCOCH_3$ | H |
| $-CH_3$ | $-C(CH_3)_2CH_2CH_2CH_2CH(CH_3)CH_2CH_2NO_2$ | H |
| -Phenyl | $-C(CH_3)_2CH_2CH_2CH_2COOCH_2CH(CH_3)C_2H_5$ | H |
| $-C_2H_5$ | $-C(CH_3)_2CH_2CH_2CH_2CONHCH_2CH(C_2H_5)C_4H_9$ | H |
| -H | $-C(CH_3)_2CH_2CH_2CH_2COOCH_2CH(CH_3)C_2H_5$ | H |
| $-CH(CH_3)_2$ | $-C(CH_3)_2CH_2CH_2CH_2COOCH_2CH(C_2H_5)C_4H_9$ | H |
| $-CH(CH_3)C_2H_5$ | $-C(CH_3)_2CH_2CH_2CH_2COO(CH_2CH_2O)_3H$ | H |
| $-CH(CH_3)_2$ | $-C(CH_3)_2CH_2CH_2CH_2COO(CH_2CH_2O)_3CH_3$ | H |
| $-CH(CH_3)_2$ | $-C(CH_3)_2CH_2CH_2CH_2CON(C_8H_{17})_2$ | H |
| $-C_2H_5$ | $-C(CH_3)_2CH_2CH_2CH_2COOCH_2P(O)(OC_4H_9)_2$ | H |
| $-C_2H_5$ | $-C(CH_3)_2CH_2CH_2CH_2COOCH_2P(O)(OCH_3)_2$ | H |
| $-C_2H_5$ | $-C(CH_3)_2CH_2CH_2CH_2COOCH_2CH(OH)CH_2P(O)(OC_2H_5)_2$ | H |
| $-CH(CH_3)_2$ | $-C(CH_3)_2CH_2CH_2CH_2COOCH_2CH(OH)C_6H_{13}$ | H |
| $-CH(CH_3)_2$ | $-C(CH_3)_2CH_2CH_2CH_2COOCH_2CH(OH)CH_2OC_8H_{17}$ | H |

Weitere Beispiele für Verbindungen der Formel I sind die folgenden Verbindungen der Formel

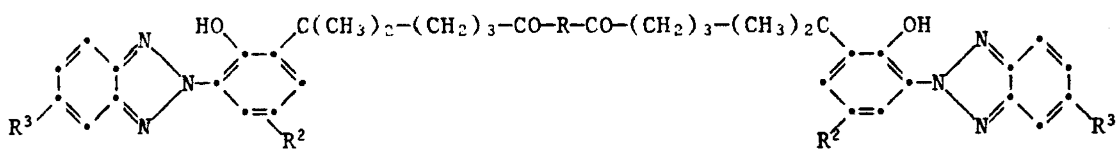

| R | $R^2$ | $R^3$ |
|---|---|---|
| $-OCH_2CH_2O-$ | $-CH(CH_3)_2$ | H |
| $-O(CH_2)_4O-$ | $-CH_3$ | Cl |
| $-OCH_2CH_2NH-$ | $-CH_3$ | H |
| $-O(CH_2CH_2O)_3-$ | $-C_2H_5$ | H |
| $-O(CH_2CH_2O)_8-$ | $-CH(CH_3)C_2H_5$ | H |
| -O- 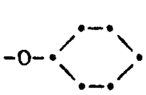 -O- | $-CH_3$<br>$-CH_3$ | H<br>H |
| $-NH-(CH_2)_6-NH-$ | $-CH(CH_3)C_2H_5$ | H |
| $-N(C_4H_9)-(CH_2)_6-N(C_4H_9)-$ | $-C_2H_5$ | H |

Weitere Beispiele für Verbindungen der Formel I sind die folgenden Verbindungen der Formel

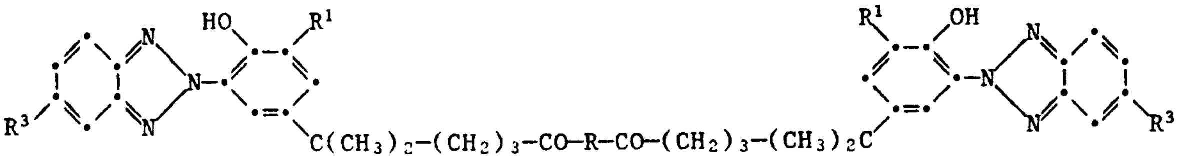

| R | $R^1$ | $R^3$ |
|---|---|---|
| $-OCH_2CH_2O-$ | $-C_2H_5$ | H |
| $-O(CH_2CH_2O)_3-$ | $-CH(CH_3)_2$ | H |
| $-O(CH_2CH_2O)_8-$ | $-CH_3$ | Cl |
| $-OCH_2CH_2NH-$ | $-CH(CH_3)C_2H_5$ | H |
| $-O-(CH_2)_6-O-$ | $-CH(CH_3)_2$ | H |

Die Verbindungen der Formel I sind neue Verbindungen.

Sie lassen sich analog anderen 2-(2-Hydroxyphenyl)-benztriazolen herstellen. Das wichtigste Herstellungsverfahren ist eine mehrstufige Synthese ausgehend von einem o-Nitroanilin, das zum entsprechenden Diazoniumsalz VII diazotiert wird. Das Diazoniumsalz VII wird mit einem substituierten Phenol VIII gekuppelt unter Bildung des Azofarbstoffes IX. Durch Reduktion von IX entsteht das Benztriazol I:

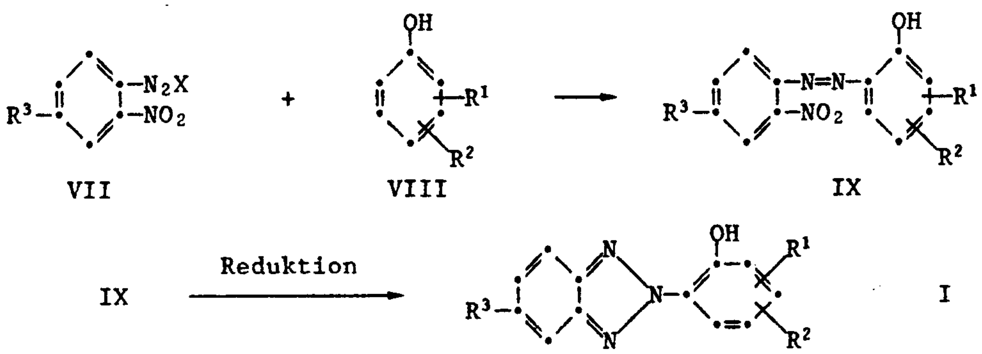


VII + VIII → IX

IX → I

Die Reduktion kann z.B. katalytisch mit $H_2$, mit Zn oder verschiedenen organischen Reduktionsmitteln erfolgen. Die entsprechenden Reduktionsverfahren sind aus der Literatur bekannt.

Substituierte Phenole der Formel VIII sind z.B. in der EP-A-106 799 beschrieben. Eine Gruppe M liegt in der Gruppe $R^1$ des Phenols VIII bereits vor. Sie kann nach Bildung des Benztriazoles I nach bekannten Methoden in andere Gruppen M umgewandelt werden. In diesem Fall werden Verbindungen der Formel I als Zwischenprodukte zur Herstellung anderer Verbindungen der Formel I verwendet.

Beispielsweise kann M zunächst eine Carboxylgruppe sein, die nach der Bildung des Benztriazols halogeniert, verestert oder amidiert wird. Ist M zunächst eine Hydroxylgruppe, so kann sie nach der Benztriazol-Bildung halogeniert oder verestert werden. Ist M zunächst ein Halogen, so kann dieses nachher z.B. in eine Alkoxygruppe, Aminogruppe, Phosphonatgruppe oder Cyangruppe überführt werden.

Wenn M eine Gruppe $-COOR^6$ ist, worin $R^6$ eine Niederalkylgruppe ist, so kann die Verbindung mit einem höheren Alkohol umgeestert werden und man erhält den entsprechenden Ester, in dem $R^6$ ein höherer Alkylrest oder ein Aralkyl- oder Cycloalkylrest ist. Der Niederalkylester ist also in diesem Fall Zwischenprodukt für den höheren Ester.

Die Verbindungen der Formel I können als Stabilisatoren für organische Materialien, insbesondere gegen deren Schädigung durch Licht, Sauerstoff und Wärme verwendet werden. Insbesondere sind sie als Lichtschutzmittel wirksam. Die zu stabilisierenden Materialien können z.B. Oele, Fette, Wachse, Kosmetika, photographische Materialien oder organische Polymere sein.

Photographische Materialien, die stabilisiert werden können, sind photographische Farbstoffe und Schichten, die solche Farbstoffe oder deren Vorprodukte enthalten, beispielsweise photographische Papiere und Filme.

Der Zusatz der erfindungsgemässen Stabilisatoren kann zu einer oder zwei oder allen drei farbempfindlichen Schichten erfolgen. Von besonderer Bedeutung ist der Zusatz zur Gelbschicht. In den Schichten befindet sich das sensibilisierte Silberhalogenid und der jeweilige Farbkuppler.

Ausserdem können die Schichten weitere Stabilisatoren und/oder sonstige Zusätze enthalten.

Die Gelbkuppler sind vorzugsweise Verbindungen der Formel A

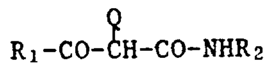 (A),

worin $R_1$ Alkyl oder Aryl ist, $R_2$ Aryl ist und Q Wasserstoff oder eine Gruppe ist, die durch Reaktion mit dem oxidierten Entwickler abgespalten werden kann.

Eine Gruppe von Gelbkupplern sind solche Verbindungen der Formel A, in denen $R_1$ t-Butyl ist und $R_2$ eine Gruppe der Formel

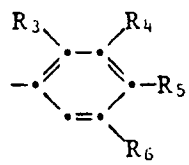

ist, worin $R_3$ Wasserstoff, Halogen, Alkyl oder Alkoxy bedeutet und $R_4$, $R_5$ und $R_6$ Wasserstoff, Halogen, Alkyl, Alkenyl, Alkoxy, Aryl, Carboxy, Alkoxycarbonyl, eine Carbamoylgruppe, eine Sulfon- oder Sulfamoylgruppe, eine Alkylsulfonamidogruppe, Acylaminogruppe, Ureidogruppe oder Aminogruppe bedeuten.

Vorzugsweise sind $R_3$ Chlor, $R_4$ und $R_5$ Wasserstoff und $R_6$ eine Acylaminogruppe. Hierzu gehören auch die Verbindungen der Formel

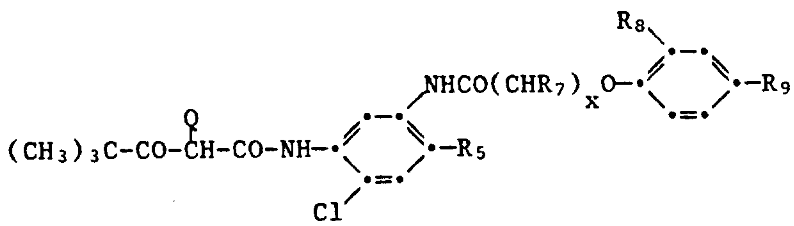 ,

worin x 0-4 ist, $R_7$ Wasserstoff oder Alkyl bedeutet, und $R_8$ und $R_9$ Alkyl sind.

Eine andere Gruppe von Gelbkupplern entspricht der Formel B

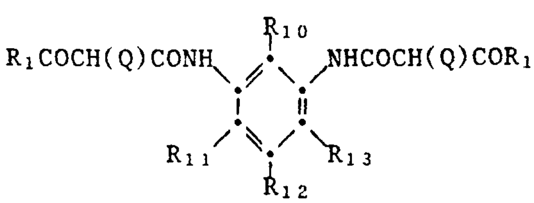 (B),

worin $R_{10}$ Wasserstoff, Halogen oder Alkoxy ist,
$R_{11}$, $R_{12}$ und $R_{13}$ Wasserstoff, Halogen, Alkyl, Alkenyl, Alkoxy, Aryl, Carboxyl, Alkoxycarbonyl, eine Carbamoylgruppe, eine Sulfongruppe, Sulfamoylgruppe, Sulfonamidogruppe, Acylaminogruppe, Ureidogruppe oder Aminogruppe bedeuten und $R_1$ und Q die oben angegebene Bedeutung haben.

Dazu gehören Verbindungen der Formel B, in denen $R_1$ t-Butyl ist, $R_{10}$ Chlor ist, $R_{11}$ und $R_{13}$ Wasserstoff sind und $R_{12}$ Alkoxycarbonyl ist.

In den Verbindungen der Formel A und B kann die Abgangsgruppe Q Wasserstoff, sein oder sie ist eine heterocyclische Gruppe

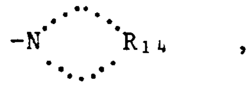

worin $R_{14}$ eine organische zweiwertige Gruppe ist, die den Ring zu einem 4-7-gliedrigen Ring ergänzt, oder Q ist eine Gruppe $-OR_{15}$, worin $R_{15}$ Alkyl, Aryl, Acyl oder ein heterocyclischer Rest ist.

Typische Beispiele für gebräuchliche Gelbkuppler sind die Verbindungen der folgenden Formeln:

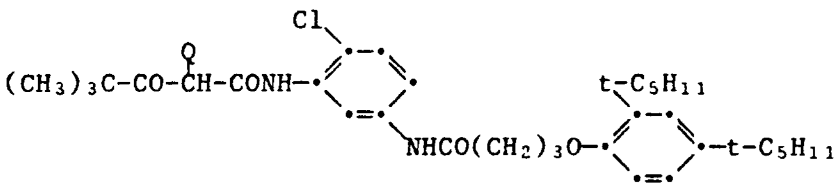

a) Q =

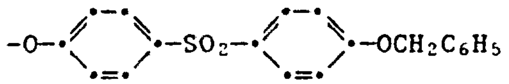

b) Q =

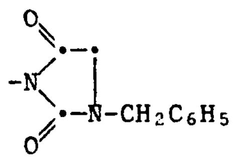

c) Q = 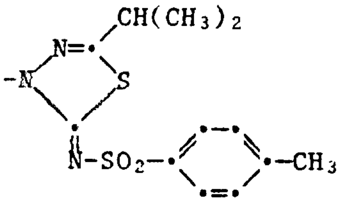

d) Q = 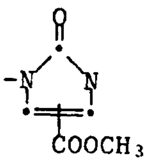

e) Q = 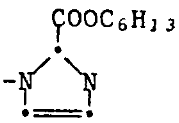

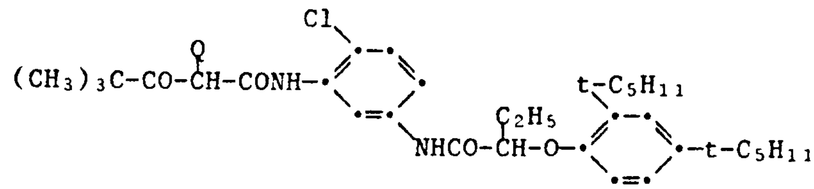

f) Q = 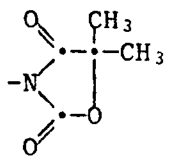

g) Q = 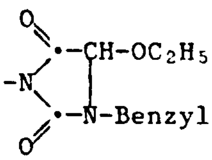

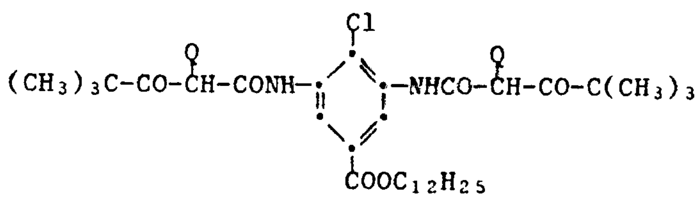

h) Q = 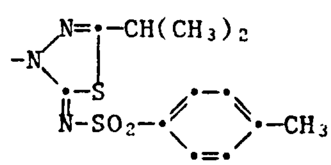

Weitere Beispiele für Gelbkuppler sind zu finden in den US-A 2,407,210, 2,778,658, 2,875,057, 2,908,513, 2,908,573, 3,227,155, 3,227,550, 2,253,924, 3,265,506, 3,277,155, 3,408,194, 3,341,331, 3,369,895, 3,384,657, 3,415,652, 3,447,928, 3,551,155, 3,582,322, 3,725,072, 3,891,445, 3,933,501,4,115,121, 4,401,752, 4,022,620, in den DE-A 1,547,868, 2,057,941, 2,162,899, 2,163,813, 2,213,461, 2,219,917, 2,261,361, 2,261,362, 2,263,875, 2,329,587, 2,414,006, 2,422,812 und in den GB-A 1,425,020 und 1,077,874.

Die Gelbkuppler werden üblicherweise in einer Menge von 0,05-2 Mol und vorzugsweise 0,1-1 Mol pro Mol Silberhalogenid verwendet.

Magentakuppler können z.B. einfache 1-Aryl-5-pyrazolone sein oder mit 5-gliederigen Heteroringen kondensierte Pyrazolderivate wie z.B. Imidazopyrazole, Pyrazolopyrazole, Pyrazolotriazole oder Pyrazolotetrazole.

Eine Gruppe von Magentakupplern sind 5-Pyrazolone der Formel C,

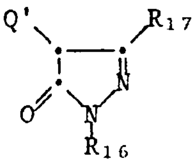

(C),

wie sie in der Britischen Patentschrift 2,003,473 beschrieben sind. Darin ist $R_{16}$ Wasserstoff, Alkyl, Aryl, Alkenyl oder eine heterocyclische Gruppe. $R_{17}$ ist Wasserstoff, Alkyl, Aryl, eine heterocyclische Gruppe, eine Estergruppe, Alkoxygruppe, Alkylthiogruppe Carboxylgruppe, Arylaminogruppe, Acylaminogruppe, (Thio)-harnstoffgruppe, (Thio)-carbamoylgruppe, Guanidinogruppe oder Sulfonamidogruppe.

Bevorzugt ist $R_{17}$ eine Gruppe

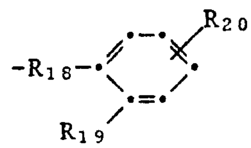

,

worin $R_{18}$ Imino, Acylamino oder Ureido ist, $R_{19}$ Wasserstoff, Halogen, Alkyl oder Alkoxy ist, $R_{20}$ Wasserstoff, Alkyl, Acylamino, Carbamoyl, Sulfamoyl, Sulfonamido, Alkoxycarbonyl, Acyloxy oder eine Urethangruppe ist.

Wenn Q' Wasserstoff ist, so ist der Magentakuppler tetraäquivalent in bezug auf das Silberhalogenid.

Typische Beispiele für diesen Typ von Magentakupplern sind Verbindungen der Formel

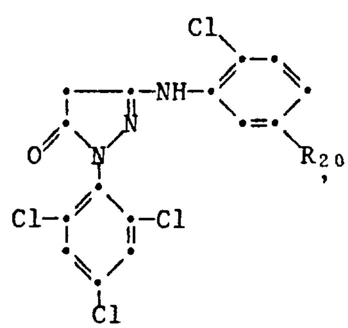

,

worin $R_{20}$ die oben genannten Bedeutungen hat.

Weitere Beispiele solcher tetraäquivalenter Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866, 3,933,500.

Wenn Q' in Formel C nicht Wasserstoff ist sondern eine Gruppe, die bei der Reaktion mit dem oxidierten Entwickler eliminert wird, so handelt es sich um einen diäquivalenten Magentakuppler. Q kann in diesem Fall z.B. Halogen oder eine über O, S oder N an den Pyrazolring gebundene Gruppe sein. Solche diäquivalente Kuppler ergeben eine höhere Farbdichte und sind reaktiver gegenüber dem oxidierten Entwickler als die entsprechenden tetraäquivalenten Magentakuppler.

Beispiele für diäquivalente Magentakuppler sind beschrieben in den US-A 3,006,579, 3,419,391, 3,311,476, 3,432,521, 3,214,437, 4,032,346, 3,701,783, 4,351,897, 3,227,554, in den EP-A-133,503, DE-A-2,944,601, JP-A-78/34044, 74/53435, 74/53436, 75/53372 und 75/122935.

Ueber ein zweiwertiges Q' können 2 Pyrazolonringe verknüpft werden und man erhält dann sogenannte Bis-Kuppler. Solche sind z.B. beschrieben in den US-A-2,632,702, US-A-2,618,864, GB-A-968,461, GB-A-786,859, JP-A-76/37646, 59/4086, 69/16110, 69/26589, 74/37854 und 74/29638.

Bevorzugt ist Y eine O-Alkoxyarylthio-Gruppe.

Wie vorstehend erwähnt, können als Magentakuppler auch mit 5-gliedrigen Heterocyclen kondensierte

Pyrazole - sogenannte Pyrazoloazole - verwendet werden. Deren Vorteile gegenüber einfachen Pyrazolen ist, dass sie Farben von grösserer Formalin-Beständigkeit und reineren Absorptionsspektren aufweisen.

Man kann sie durch die allgemeinen Formeln $D_1$ und $D_2$ darstellen,

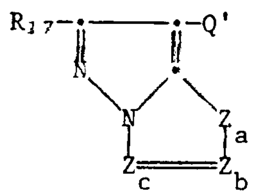 ($D_1$) 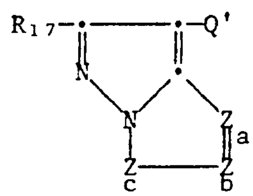 ($D_2$) ,

worin $Z_a$, $Z_b$ und $Z_c$ die Ergänzungen zu einem 5-gliedrigen Ring bedeuten, der bis zu 4 Stickstoffatome enthalten kann. Die Verbindungen können demgemäss Pyrazolo-imidazole, Pyrazolo-pyrazole, Pyrazolo-triazole oder Pyrazolo-tetrazole sein. $R_{17}$ und Q' haben dieselben Bedeutungen wie in Formel C.

Pyrazolo-tetrazole sind beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-119,860, EP-A-173,256, EP-A-178,789, EP-A-178,788 und in Research Disclosure 84/24,624.

Weitere Pyrazoloazol-Magentakuppler sind beschrieben in: JP-A-86/28,947, JP-A-85/140,241, JP-A-85/262,160, JP-A-85/213,937, EP-A-177,765, EP-A-176,804, EP-A-170,164, EP-A-164,130, EP-A-178,794, DE-A-3,516,996, DE-A-3,508,766 und Research Disclosure 81/20919, 84/24531 und 85/25758.

Cyankuppler können z.B. Derivate von Phenol, von 1-Naphthol oder von Pyrazolochinazolon sein. Bevorzugt sind Strukturen der Formel E,

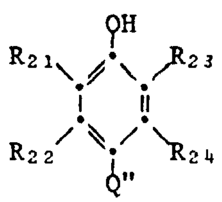 (E),

worin $R_{21}$, $R_{22}$, $R_{23}$ und $R_{24}$ Wasserstoff, Halogen,Alkyl, Carbamoyl, Amido, Sulfonamido, Phosphoramido oder Ureido sind. $R^{21}$ ist vorzugsweise H oder Cl, $R_{22}$ ist vorzugsweise eine Alkyl- oder Amidogruppe. $R_{23}$ ist vorzugsweise eine Amidogruppe und $R_{24}$ ist vorzugsweise Wasserstoff. Q" ist Wasserstoff oder eine Abgangsgruppe, die bei der Reaktion mit dem oxidierten Entwickler abgespalten wird. Eine ausführliche Aufzählung von Cyankupplern ist im US-A-4,456,681 zu finden.

Beispiele von gebräuchlichen Cyankupplern sind die folgenden:

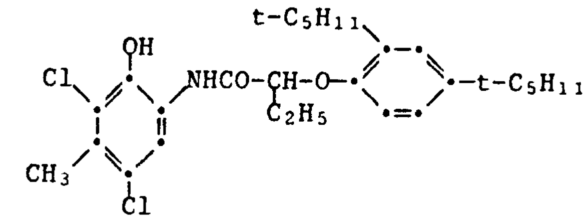

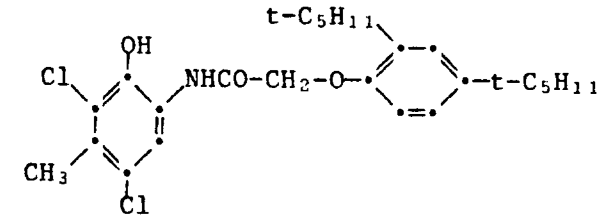

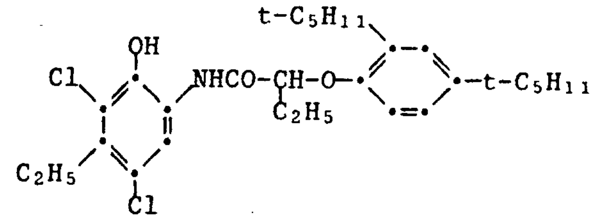

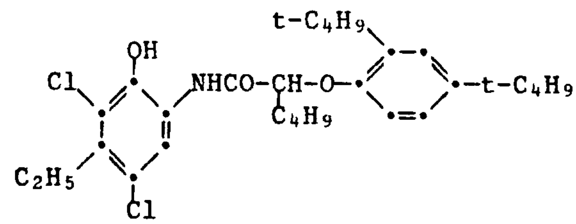

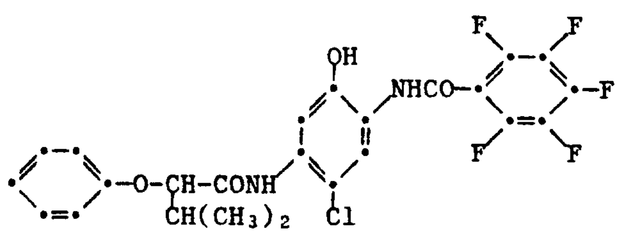

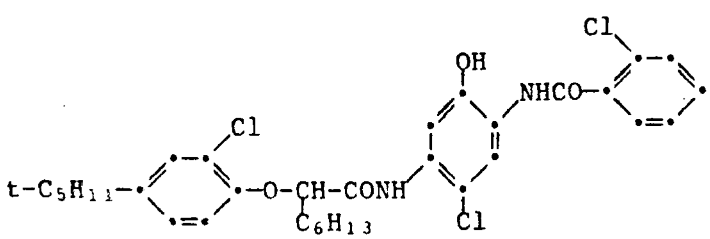

Weitere Beispiele von Cyankupplern sind in folgenden US-A zu finden: 2,369,929, 2,423,730, 2,434,272, 2,474,293, 2,521,908, 2,698,794, 2,706,684, 2,772,162, 2,801,171, 2,895,826, 2,908,573, 3,034,892, 3,046,129, 3,227,550, 3,253,294, 3,311,476, 3,386,301, 3,419,390, 3,458,315, 3,476,560, 3,476,563, 3,516,831, 3,560,212, 3,582,322, 3,583,971, 3,591,383, 3,619,196, 3,632,347, 3,652,286, 3,737,326, 3,758,308, 3,839,044, 3,880,661, 4,004,929, 4,124,396, 4,333,999, 4,463,086, 4,456,681.

Die für farbfotographische Materialien üblicherweise verwendeten Farbentwickler sind p-Dialkylaminoaniline. Beispiele hierfür sind 4-Amino-N,N-diethylanilin, 3-Methyl-4-amino-N,N-diethylanilin, 4-Amino-N-ethyl-N-α-hydroxyethylanilin, 3-Methyl-4-amino-N-ethyl-N-α-hydroxyethylanilin, 3-Methyl-4-amino-N-ethyl-N-α-methansulphonamidoethylanilin, 3-Methyl-4-amino-N-ethyl-N-α-methoxyethyl-anilin, 3-α-Methansulphonamidoethyl-4-amino-N,N-diethylanilin, 3-Methoxy-4-amino-N-ethyl-N-α-hydroxyethylanilin, 3-Methoxy-4-amino-N-ethyl-N-α-methoxyethylanilin, 3-Acetamido-4-amino-N,N-diethylanilin, 4-Amino-N,N-dimethylanilin, N-Ethyl-N-α-[α'-(α''-methoxyethoxy)ethoxy]ethyl-3-methyl-4-aminoanilin, N-Ethyl-N-α-(α'-methoxyethoxy)ethyl-3-methyl-4-aminoanilin sowie die Salze solcher Verbindungen, wie z.B. Sulfate, Hydrochloride oder Toluolsulfonate.

Die erfindungsgemässen Stabilisatoren können allein oder zusammen mit dem Farbkuppler und gegebenenfalls weiteren Zusätzen in das farbphotographische Material eingearbeitet werden, indem man sie in hochsiedenden organischen Lösungsmitteln vorlöst. Vorzugsweise verwendet man Lösungsmittel, die höher als 160 °C sieden. Typische Beispiele solcher Lösungsmittel sind die Ester von Phthalsäure, Phosphorsäure, Zitronensäure, Benzoesäure oder von Fettsäuren, sowie Alkylamide und Phenole.

Meist verwendet man zusätzlich noch ein niedrig siedendes Lösungsmittel, um das Einarbeiten der Zusätze in das farbphotographische Material zu erleichtern. Beispiele für solche Lösungsmittel sind Ester wie z.B. Ethylacetat, Alkohole wie z.B. Butanol, Ketone wie z.B. Methyl-isobutylketon, Chlorkohlenwasserstoffe wie z.B. Methylenchlorid, oder Amide wie z.B. Dimethylformamid. Sind die Zusätze selbst flüssig, so kann man sie auch ohne Zuhilfenahme von Lösungsmitteln in das Photomaterial einarbeiten.

Weitere Details über verwendbare hochsiedende Lösungsmittel sind in den folgenden Veröffentlichungen zu finden.

Phosphate: GB-A-791,219, BE-A-755,248, JP-A-76/76739, 78/27449, 78/218,252, 78/97573, 79/148,113, 82/216,177, 82/93323 und 83/216,177.

Phthalate: GB-A-791,219, JP-A-77/98050, 82/93322, 82/216,176, 82/218,251, 83/24321, 83/45699, 84/79888.

Amide: GB-A-791,219, JP-A-76/105,043, 77/13600, 77/61089, 84/189,556, US-A-928,741.

Phenole: GB-A-820,329, FR-A-1,200,657, JP A-69/69946, 70/3818, 75/123,026, 75/82078, 78/17914, 78/21166, 82/212,114 und 83/45699.

Andere sauerstoffhaltige Verbindungen: US-A-3,748,141, 3,779,765, JP-A-73/75126, 74/101,114, 74/10115, 75/101,625, 76/76740, 77/61089 und BE-A-826,039.

Sonstige Verbindungen: JP-A-72/115,369, 72/130,258, 73/127,521, 73/76592, 77/13193, 77/36294, 79/95233 und Research Disclosure 82/21918.

Die Menge von hochsiedendem Lösungsmittel liegt z.B. im Bereich von 0,1 bis 300 %, vorzugsweise 10 bis 100 %, bezogen auf den Farbkuppler.

Die photographischen Schichten können ferner Farbschleier-Inhibitoren enthalten. Diese verhindern das Entstehen von Farbschleiern, wie sie beispielsweise durch Reaktion des Kupplers mit unabsichtlich oxidiertem Entwickler oder mit Nebenprodukten des Farbbildungsprozesses entstehen. Solche Farbschleierinhibitoren sind meist Hydrochinonderivate, können aber auch Derivate von Aminophenolen, von Gallussäure oder von Ascorbinsäure sein. Typische Beispiele hierfür sind in folgenden Veröffentlichungen zu finden: US-A-2,360,290, 2,336,327, 2,403,721, 2,418,613, 2,675,314, 2,701,197, 2,704,713, 2,728,659, 2,732,300, 2,735,365; EP-A-124,877; JP-A-75/92988, 75/92989, 75/93928, 75/110,337 und 77/146,235.

Die photographischen Schichten können auch sogenannte DIR-Kuppler (DIR bedeutet Development Inhibition Release) enthalten, die mit dem oxidierten Entwickler farblose Verbindungen ergeben. Sie werden zugesetzt zur Verbesserung der Schärfe und Körnigkeit der Farbbilder.

Die photographischen Schichten können auch UV-Absorber enthalten. Diese filtern das UV-Licht aus und schützen damit die Farbstoffe, die Kuppler oder sonstige Komponenten gegen Lichtabbau. Beispiele für solche UV-Absorber sind 2-(2-Hydroxyphenyl)-benztriazole, 2-Hydroxybenzophenone, Salicylsäureester, Acrylnitrilderivate oder Thiazoline. Solche UV-Absorber sind z.B. in folgenden Veröffentlichungen näher erläutert: US-A-3,314,794, 3,352,681, 3,705,805, 3,707,375, 4,045,229, 3,700,455, 3,533,794, 3,698,907, 3,705,805, 3,738,837 und JP-A-71/2784. Bevorzugte UV-Absorber sind die 2-(2-Hydroxyphenol)-benztriazole.

Die photographischen Schichten können auch phenolische Verbindungen enthalten, die als Lichtschutzmittel für das Farbbild sowie als Mittel gegen Farbschleier wirken. Sie können in einer lichtempfindlichen Schicht (Farbschicht) oder in einer Zwischenschicht enthalten sein, allein oder zusammen mit anderen Additiven. Solche Verbindungen sind z.B. in den folgenden Veröffentlichungen näher beschrieben:

US-A-3,700,455, 3,591,381, 3,573,052, 4,030,931, 4,174,220, 4,178,184, 4,228,235, 4,279,990, 4,346,165, 4,366,226, 4,447,523, 4,528,264, 4,581,326, 4,562,146, 4,559,297, GB-A-1,309,277, 1,547,302, 2,023,862, 2,135,788, 2,139,370, 2,156,091; DE-A-2,301,060, 2,347,708, 2,526,468, 2,621,203, 3,323,448; DD-A-200,691, 214,468; EP-A-106,799, 113,124, 125,522, 159,912, 161,577, 164,030, 167,762, 176,845; JP-A-74/134,326, 76/127,730, 76/30462, 77/3822, 77/154,632, 78/10842, 79/48535, 79/70830, 79/73032, 79/147,038, 79/154,325, 79/155,836, 82/142,638, 83/224,353, 84/5246, 84/72443, 84/87456, 84/192,246,

84/192,247, 84/204,039, 84/204,040, 84/212,837, 84/220,733, 84/222,836, 84/228,249, 86/2540, 86,8843, 86/18835, 86/18836, 87/11456, 87/42245, 87/62157, 86/6652 sowie in Research Disclosure 79/17804.

Die photographischen Schichten können auch gewisse Phosphor-III-Verbindungen, insbesondere Phosphite und Phosphonite, enthalten. Diese fungieren als Lichtschutzmittel für die Farbbilder sowie als Dunkellager-Stabilisator für Magentakuppler. Man setzt sie vorzugsweise den hochsiedenden Lösungsmitteln zu, zusammen mit dem Kuppler. Solche Phosphor-III-Verbindungen sind z.B. in den folgenden Veröffentlichungen näher beschrieben: US-A-4,407,935, US-A-4,436,811, EP-A-181,289, JP-A-73/32728, JP-A-76/1420 und JP-A-55/67741.

Die photographischen Schichten können auch metallorganische Komplexe enthalten, die Lichtschutzmittel für die Farbbilder sind, insbesondere für die Magenta-Farbstoffe. Solche Verbindungen und deren Kombination mit anderen Additiven sind z.B. in folgenden Veröffentlichungen näher beschrieben: US-A-4,050,938, 4,239,843, 4,241,154, 4,242,429, 4,241,155, 4,242,430, 4,273,854, 4,246,329, 4,271,253, 4,242,431, 4,248,949, 4,245,195, 4,268,605, 4,246,330, 4,269,926, 4,245,018, 4,301,223, 4,343,886, 4,346,165, 4,590,153; JP-A-81/167,138, 81/168,652, 82/30834, 82/161,744; EP-A-137,271, 161,577, 185,506; DE-A-2,853,865.

Die photographischen Schichten können auch Hydrochinonverbindungen enthalten. Diese wirken als Lichtschutzmittel für die Farbkuppler und für die Farbbilder sowie als Abfänger von oxidiertem Entwickler in Zwischenschichten. Sie werden vor allem in der Magentaschicht verwendet. Solche Hydrochinon-Verbindungen und deren Kombinationen mit anderen Additiven sind z.B. in folgenden Veröffentlichungen näher beschrieben:

US-A-2,360,290, 2,336,327, 2,403,721, 2,418,613, 2,675,314, 2,701,197, 2,710,801, 2,732,300, 2,728,659, 2,735,765, 2,704,713, 2,937,086, 2,816,028, 3,582,333, 3,637,393, 3,700,453, 3,960,570, 3,935,016, 3,930,866, 4,065,435, 3,982,944, 4,232,114, 4,121,939, 4,175,968, 4,179,293, 3,591,381, 3,573,052, 4,279,990, 4,429,031, 4,346,165, 4,360,589, 4,346,167, 4,385,111, 4,416,978, 4,430,425, 4,277,558, 4,489,155, 4,504,572, 4,559,297, FR-A-885,982; GB-A-891,158, 1,156,167, 1,363,921, 2,022,274, 2,066,975, 2,071,348, 2,081,463, 2,117,526, 2,156,091; DE-A-2,408,168, 2,726,283, 2,639,930, 2,901,520, 3,308,766, 3,320,483, 3,323,699; DD-A-216,476, 214,468, 214,469, EP-A-84290, 110,214, 115,305, 124,915, 124,877, 144,288, 147,747, 178,165, 161,577; JP-A-75/33733, 75/21249, 77/128,130, 77/146,234, 79/70036, 79/133,131, 81/83742, 81/87040, 81/109,345, 83/134,628, 82/22237, 82/112,749, 83/17431, 83/21249, 84/75249, 84/149,348, 84/182,785, 84/180,557, 84/189,342, 84/228,249, 84/101,650, 79/24019, 79/25823, 86/48856, 86/48857, 86/27539, 86/6652, 86/72040, 87/11455, 87/62157, sowie in Research Disclosure 79/17901, 79/17905, 79/18813, 83/22827 und 84/24014.

Die photographischen Schichten können auch Derivate von Hydrochinonethern enthalten. Diese Verbindungen wirken als Lichtschutzmittel und sind besonders geeignet zur Stabilisierung von Magenta-Farbstoffen. Solche Verbindungen und deren Kombination mit anderen Additiven sind z.B. in folgenden Veröffentlichungen näher beschrieben:

US-A 3,285,937, 3,432,300, 3,519,429, 3,476,772, 3,591,381, 3,573,052, 3,574,627, 3,573,050, 3,698,909, 3,764,337, 3,930,866, 4,113,488, 4,015,990, 4,113,495, 4,120,723, 4,155,765, 4,159,910, 4,178,184, 4,138,259, 4,174,220, 4,148,656, 4,207,111, 4,254,216, 4,314,011, 4,273,864, 4,264,720, 4,279,990, 4,332,886, 4,436,165, 4,360,589, 4,416,978, 4,385,111, 4,459,015, 4,559,297; GB-A 1,347,556, 1,366,441, 1,547,392, 1,557,237, 2,135,788; DE-A 3,214,567; DD-214,469, EP-A 161,577, 167,762, 164,130, 176,845; JP-A 76/123,642, 77/35633, 77/147,433, 78/126, 78/10430, 78/53321, 79/24019, 79/25823, 79/48537, 79/44521, 79/56833, 79/70036, 79/70830, 79/73032, 79/95233, 79/145,530, 80/21004, 80/50244, 80/52057, 80/70840, 80/139,383, 81/30125, 81/151,936, 82/34552, 82/68833, 82/204,036, 82/204,037, 83/134,634, 83/207,039, 84/60434, 84/101,650, 84/87450, 84/149,348, 84/182,785, 86/72040, 87/11455, 87/62157, 87/63149, 86/2151, 86/6652, 86/48855 sowie in Research Disclosure 78/17051.

Beispiele von organischen Polymeren, die mit den erfindungsgemässen Benztriazolen stabilisiert werden können, sind die folgenden Klassen von Polymeren.

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben

mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/ Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner

mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Von besonderer Bedeutung ist die Verwendung in Lacken. Dies können pigmentierte oder unpigmentierte Lacke sein, sie können kalt- oder warmhärtend oder physikalisch trocknend sein. Die Lacke können ein organisches Lösungsmittel enthalten oder sie können lösungsmittelfrei sein oder wässrige Lacke sein.

Die Stabilisatoren werden den organischen Materialien in einer Konzentration von 0,01 bis 5 Gew.-%, berechnet auf das zu stabilisierende Material, zugesetzt. Es können auch Gemische von zwei oder mehreren Verbindungen der Formel I verwendet werden.

Die Verbindungen der Formel I können zusammen mit anderen Stabilisatoren verwendet werden. Dies können z.B. Antioxidantien oder Lichtschutzmittel sein, oder Co-Stabilisatoren, wie z.B. organische Phosphite oder Phosphonate.

Beispiele hierfür sind die folgenden Klassen von Stabilisatoren.

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Di-phenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-($\alpha$-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-($\alpha$-methylbenzyl)-4-nonylphenol], 2,2'-Methylen-bis-[6-($\alpha$,$\alpha$-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat],Bis-(3-tert.butyl-4-hydroxy-5-methylphenyl)-dicyclopentadien, Bis-[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiolterephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der $\beta$-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der $\beta$-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.9. Ester der $\beta$-(3,5-Dicyclohexyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der $\beta$-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-($\alpha$,$\alpha$-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. $\alpha$-Cyan-ß,ß-diphenylacrylsäure-ethylester bzw. -isooctylester, $\alpha$-Carbomethoxy-zimtsäuremethylester, $\alpha$-Cyano-$\beta$-methyl-p-methoxy-zimtsäuremethylester bzw. -butylester, $\alpha$-Carbomethoxy-p-methoxy-zimtsäure-methylester, N-($\beta$-Carbomethoxy-$\beta$-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethylpiperazinon).

2.7. Oxalsäurediamide, wie z.B. 4,4'-Di-octyloxy-oxanilid, 2,2'-Dioctyloxy-5,5'-di-tert.butyl-oxanilid, 2,2'-Didodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyloxanilid, Gemische von o-und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der $\beta$-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-($\beta$-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

Handelt es sich um die Stabilisierung von Kunststoffen, so können als weitere Zusätze z.B. Antistatika, Weichmacher, Gleitmittel, Flammschutzmittel, Treibmittel, Metalldesaktivatoren, Füllstoffe, Verstärkungsmittel oder Pigmente verwendet werden. Handelt es sich um die Stabilisierung von Lacken, so können als weitere Zusätze z.B. Verlaufhilfsmittel, Härtungsbeschleuniger, Verdicker oder Haftverbesserer verwendet werden. Handelt es sich um die Stabilisierung von photographischen Materialien, so können als weitere Zusätze z.B. Farbschleier-Inhibitoren, Hydrochinon-Verbindungen oder Lösungsmittel verwendet werden.

Gegenstand der Erfindung sind daher auch organische Materialien, die als Stabilisator mindestens eine Verbindung der Formel I enthalten.

Die organischen Materialien sind vorzugsweise Kunststoffe, Lacke oder photographische Schichten. Die so stabilisierten Materialien enthalten 0,01 bis 5 Gew.-% des Stabilisatoren der Formel I.

Die folgenden Beispiele beschreiben die Erfindung näher ohne dass die Erfindung auf die Beispiele beschränkt ist. Darin bedeuten Teile Gewichtsteile und % Gewichts-%, sofern nicht anders angegeben.

## Beispiel 1: Herstellung der Benztriazole durch Diazotierung, Kupplung und Reduktion

### a) Diazotierung

In 50 ml 37%ige Salzsäure werden bei Raumtemperatur 0,17 Mol fein pulverisiertes 2-Nitroanilin eingetragen. Die gelbe Suspension erwärmt sich auf 30-35 °C. Sie wird mit Eis auf 0-5 °C gekühlt. Bei dieser Temperatur wird innert 50 Min. eine ca. 30%ige wässerige Lösung von 0,18 Mol Natriumnitrit unter Niveau zugetropft. Es entsteht eine braune Lösung, die während einer Stunde ausgerührt wird. Nach Zugabe von 1 g Aktivkohle wird über Kieselerde filtriert. Zuvor kann überschüssige salpetrige Säure mit Sulfaminsäure beseitigt werden. Nach dem Filtrieren erhält man eine hellgelbe klare Lösung die in b) weiterverwendet wird.

### b) Kupplung

0,15 Mol 2-(1,1-Dimethyl-4-methoxycarbonyl-butyl)-4-methylphenol werden in einer Lösung aus 0,25 Mol Natriumhydroxid in 100 ml Wasser und wenig Isopropanol auf 70-80 °C erhitzt und ca. 30 Min. gerührt. Die Estergruppe verseift dabei und das Phenol geht in Lösung. Die Lösung wird auf 0-5 °C gekühlt und tropfenweise mit der obigen Diazoniumsalzlösung versetzt. Zutropfzeit ca. 1 Stunde. Der pH der Lösung wird bei 11-12 gehalten. Leichtes Schäumen wird durch Zugabe von 1 ml n-Octanol unterbunden. Nach dem Zutropfen erhält man eine dunkelviolette Azofarbstofflösung. Diese wird als solche weiterverwendet.

### c) Reduktion

Die Farbstofflösung wird nochmals mit 1,5 Mol Natriumhydroxid in 200 ml Wasser und 50 ml Ethylcellulose versetzt und auf 80 °C erwärmt. Bei dieser Temperatur tropft man zur dunkelvioletten Lösung innert 20 Min. 0,2 Mol Hydrazinhydrat zu. Unter deutlicher Stickstoffabspaltung wird die Reaktionslösung dunkelbraun. Nach 2-stündigem Ausrühren bei 80 °C wird auf 40-50 °C gekühlt und portionenweise 0,3 Mol

Zinkstaub eingetragen. Dabei tritt eine exotherme Reaktion ein und die Lösung färbt sich grau-grün. Man lässt ca. 2-3 Stunden bei 70-80 °C ausrühren und filtriert dann heiss über Kieselerde. Man erhält ein klares braunes Filtrat, das unter Kühlung mit konzentrierter Salzsäure (37%ig) kongosauer gestellt wird. Das dabei ausfallende braune Rohprodukt wird durch Umkristallisation oder mittels Säulenchromatographie (Kieselgel) gereinigt oder roh weiterverarbeitet (siehe d).

d) Veresterung

0,15 Mol der rohen Benztriazolcarbonsäure werden in 250 ml Methanol zum Rückfluss erwärmt und bei dieser Temperatur (~65 °C) werden 2 ml konz. Schwefelsäure zugetropft. Nach ca. 3 Stunden Rückfluss zeigt das DC keine Benztriazolcarbonsäure mehr. Man erhält nach dem Eindampfen der Methanollösung das rohe 2-[2-Hydroxy-3-(1,1-dimethyl-4-methoxycarbonyl-butyl)-5-methyl]-benztriazol als bräunliches Produkt. Das Produkt lässt sich aus Methanol oder aus Benzin (80-100 °C) umkristallisieren. Smp. 88-90 °C (Verbindung Nr. 1).

In analoger Weise lassen sich die in Tabelle 1 aufgeführten Verbindungen aus dem o-Nitrophenyldiazoniumsalz und den entsprechenden Phenolen herstellen.

Tabelle 1

| Verbindung Nr. | Formel | Physikal. Eigenschaften Analyse |
|---|---|---|
| 2 | 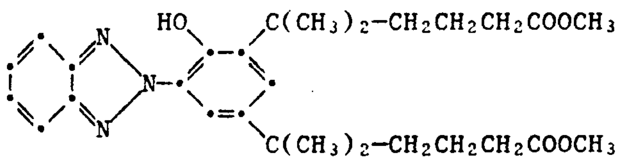 | Oel<br>N = 8,43 %,<br>ber. 8,48 % |
| 3 | 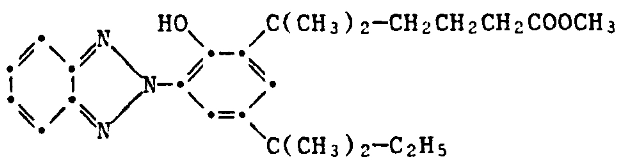 | Fp. 77-80°C<br>(aus Methanol) |
| 4 | 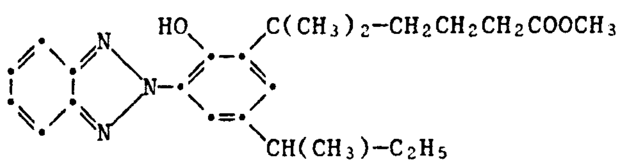 | Oel<br>N = 10,14 %,<br>ber. 10,26 % |
| 5 | 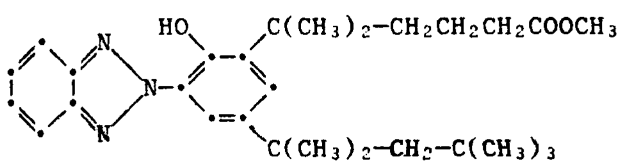 | Oel<br>N = 8,78 %,<br>ber. 9,02 % |
| 6 | 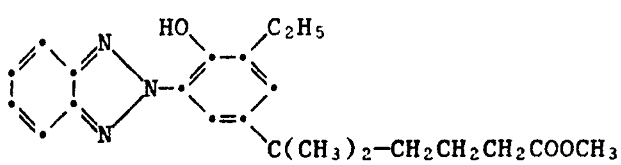 | Fp. 51-53°C<br>(aus Methanol) |
| 7 | 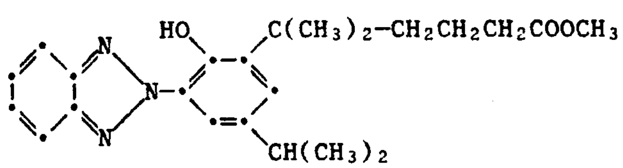 | Oel<br>N = 10,49 %,<br>ber. 10,62 % |
| 8 | 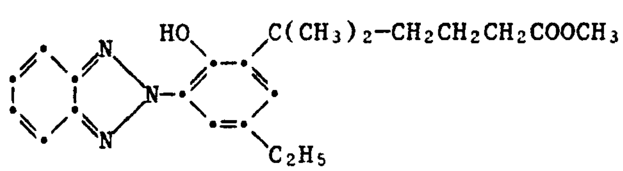 | Fp. 81-82°C<br>(aus Methanol) |

Tabelle 1 (Fortsetzung)

| Verbindung Nr. | Formel | Physikal. Eigenschaften Analyse |
|---|---|---|
| 9 | 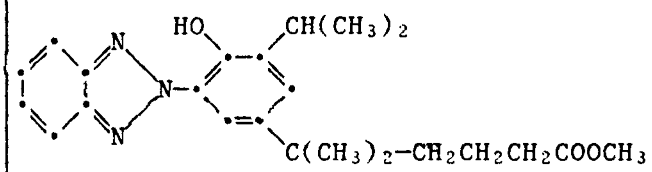 | Oel<br>N = 10,4 %,<br>ber. 10,6 % |
| 10 | 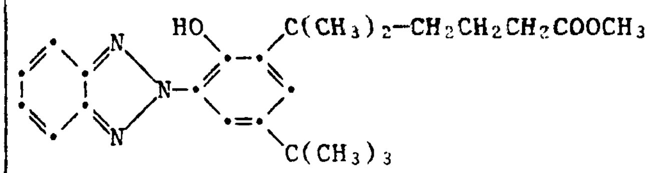 | Oel |
| 11 | 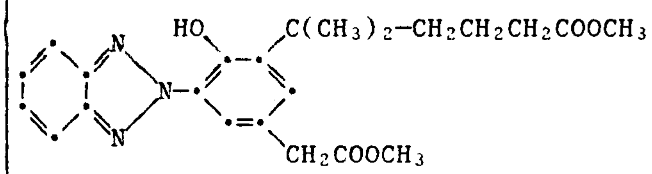 | Oel<br>N = 9,50 %,<br>ber. 9,88 % |
| 12 | 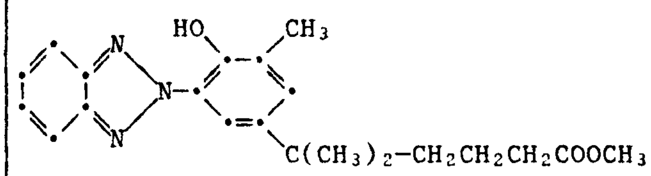 | Fp. 92-93°C<br>(aus Methanol) |
| 13 | 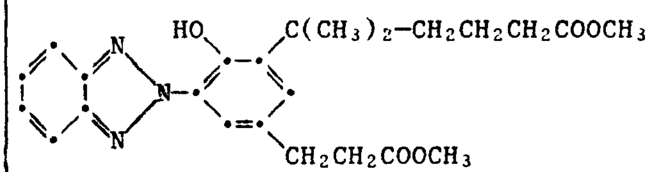 | Fp. 82-84°C<br>(aus Methanol) |
| 14 | 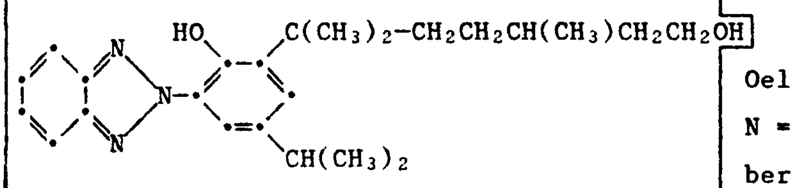 | Oel<br>N = 9,65 %<br>ber. 10,26 % |
| 15 | 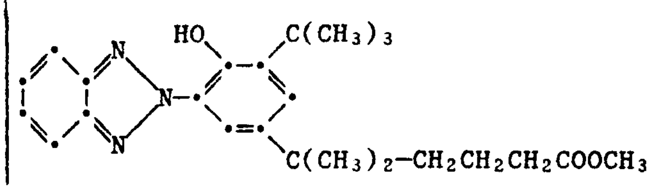 | Fp. 79-81°C<br>(aus Methanol) |

Tabelle 1 (Fortsetzung)

| Verbindung Nr. | Formel | Physikal. Eigenschaften Analyse |
|---|---|---|
| 16 | 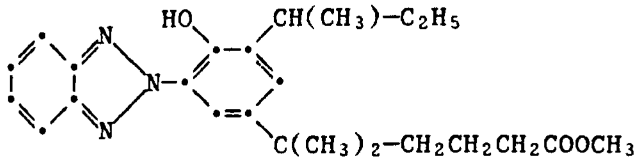 | Oel<br>N = 10,33 %,<br>ber. 10,26 % |
| 17 | 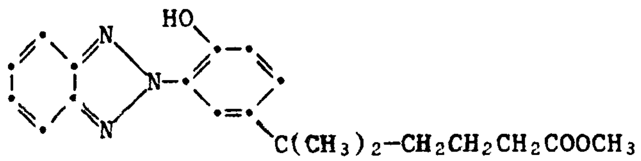 | Fp. 66-68°C<br>(aus Methanol) |
| 18 | 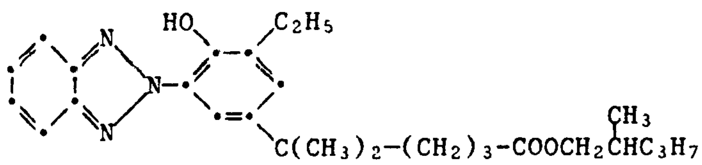 | Oel<br>N = 9,30 %<br>ber. 9,30 % |
| 19 | 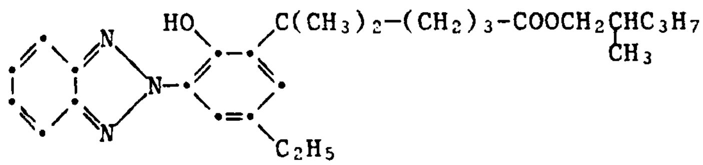 | Oel<br>N = 9,37 %<br>ber. 9,30 % |
| 20 | 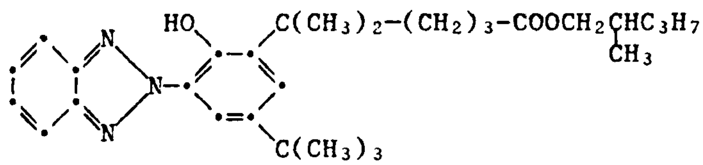 | Oel<br>N = 8,72 %<br>ber. 8,76 % |
| 21 | 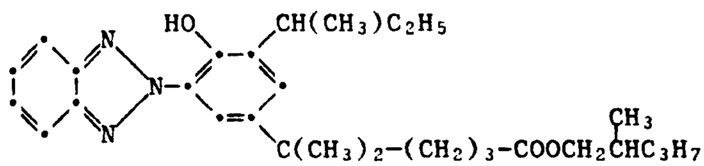 | Oel<br>N = 8,77 %<br>ber. 8,76 % |
| 22 | 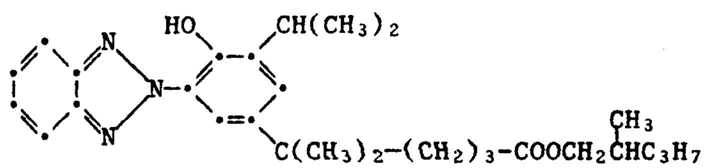 | Oel<br>N = 9,03 %<br>ber. 8,58 % |

Beispiel 2: Umesterung der Benztriazole

In einem 250 ml Sulfierkolben mit Rührer, Destillationsaufsatz, Thermometer und Stickstoff-Spülung wird ein Gemisch aus 18,4 g 2-[2-Hydroxy-3-(1,1-dimethyl-4-methoxycarbonyl-butyl)-5-methyl)-phenyl]-benztriazol (Verbindung Nr. 1), 3,4 g n-Octanol, 3,4 g 2-Ethylhexanol und 0,12 g Dibutylzinnoxid auf 150 °C erwärmt. Bei dieser Temperatur wird Toluol zugetropft und zusammen mit dem gebildeten Methanol laufend abdestilliert. Nach drei Stunden zeigt ein Dünnschicht-Chromatogramm, dass im Reaktionsgemisch kein Methylester mehr vorhanden ist. Das Reaktionsgemisch wird nach dem Abkühlen in Methylenchlorid gelöst, die Lösung wird über Kieselerde filtriert und im Vakuum eingedampft. Man erhält ein gelbes Oel (Verbindung Nr. 23), das ein 1:1-Gemisch ist aus 2-[2-Hydroxy-3-(1,1-dimethyl-4-n-octyloxycarbonyl-butyl)-5-methyl)-phenyl]-benztriazol und 2-[2-Hydroxy-3-(1,1-dimethyl-4-[2-ethylhexyloxy]carbonyl-5-methyl)-phenyl]-benztriazol.

| | | | |
|---|---|---|---|
| Analyse: Gef. | C = 72,33 | H = 8,26 | N = 9,27 % |
| Ber. | C = 72,13 | H = 8,44 | N = 9,02 % |

In analoger Weise werden die in Tabelle 2 aufgeführten Verbindungen hergestellt.

Tabelle 2

| Verbindung Nr. | Formel | Physikal. Eigenschaften Analyse |
|---|---|---|
| 24 | 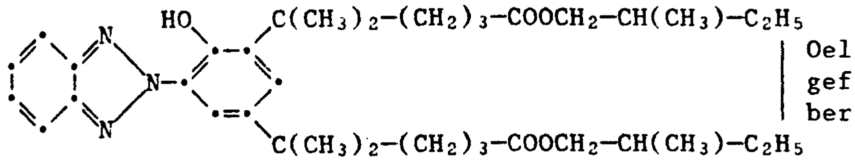<br>hergestellt aus Verbindung Nr. 2 | Oel<br>gef. 7,07 % N<br>ber. 6,91 % N |
| 25 | 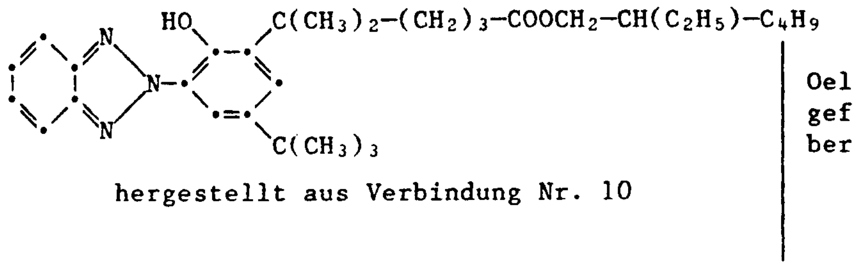<br>hergestellt aus Verbindung Nr. 10 | Oel<br>gef. 7,95 % N<br>ber. 8,28 % N |
| 26 | 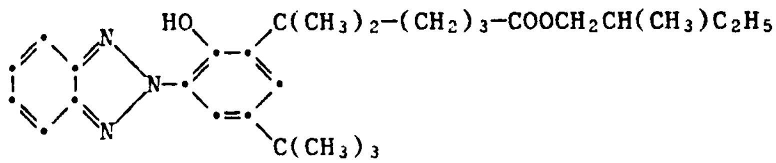<br>hergestellt aus Verbindung Nr. 10 | Oel<br>gef. 8,92 % N<br>ber. 9,02 % N |
| 27 | 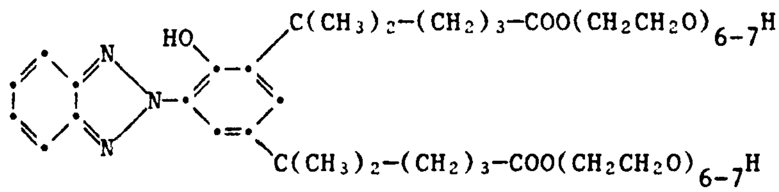<br>aus Verbindung Nr. 2 und Polyethylenglykol 300 | Oel<br>N = 4,51 %<br>ber. 4,22 % |
| 28 | 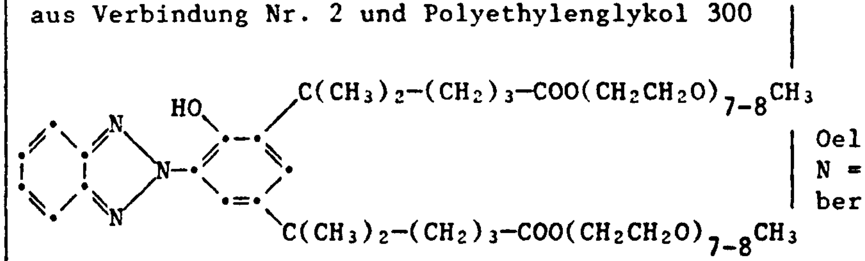<br>aus Verbindung Nr. 2 und Polyethylenglykol 350-monomethylether | Oel<br>N = 4,00 %<br>ber. 4,10 % |

Tabelle 2 (Fortsetzung)

| Verbindung Nr. | Formel | Physikal. Eigenschaften Analyse |
|---|---|---|
| 29 | 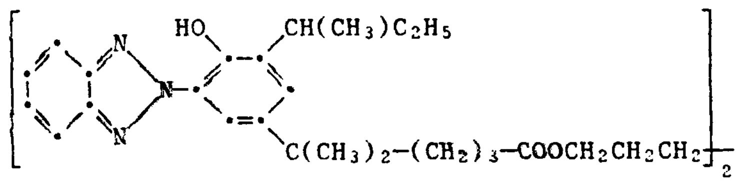 aus Verbindung Nr. 21 und Hexandiol-1,6 | Harz N = 9,66 % ber. 9,62 % |
| 30 | 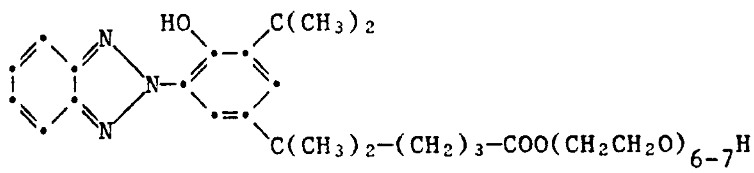 aus Verbindung Nr. 9 und Polyethylenglykol 300 | Oel N = 6,44 % ber. 6,09 % |

Beispiel 3: Veresterung der Benztriazolcarbonsäuren mit Epoxiden

0,05 Mol 2-[2-Hydroxy-3-ethyl-5-(1,1-dimethyl-4-carboxybutyl)-phenyl]-benztriazol werden in 100 ml Toluol gelöst. Man erwärmt die Lösung auf 80 °C und fügt 0,055 Mol Butyl-glycidyl-ether und 0,005 Mol Dimethylbenzylamin als Katalysator zu. Nach 12 h Rühren bei 80 °C wird die Lösung im Vakuum eingedampft. Der ölige Rückstand wird über Kieselgel chromatographiert. Man erhält die Verbindung

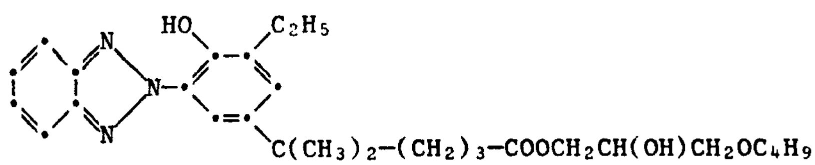

als farbloses Harz (Verbindung Nr. 31).
Analyse N ber. 8,51 % gef. 8,44 %
In analoger Weise erhält man bei Verwendung von Butyloxiran (Hexen-1-oxid) die Verbindung

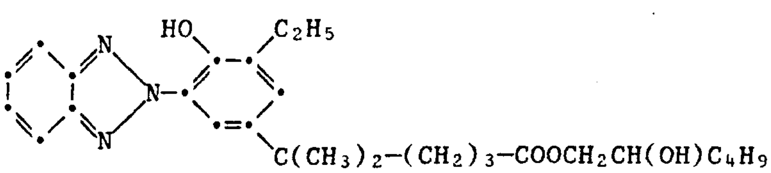

als gelbliches Oel (Verbindung Nr. 32).

Beispiel 4: Veresterung und Amidierung via Carbonsäurechlorid

a) Herstellung des Carbonsäurechlorides

0,1 Mol 2-[2-Hydroxy-3-ethyl-5-(1,1-dimethyl-4-carboxybutyl)-phenyl]-benztriazol wird in 200 ml Hexan dispergiert. Nach Zusatz von 0,5 ml Dimethylformamid werden 0,12 Mol Thionylchlorid unter Rühren langsam zugetropft. Es bildet sich eine kräftige Gasentwicklung ($HCl/SO_2$-Gemisch) und die Temperatur steigt bis 35 °C. Nach Beendigung des Zutropfens wird langsam bis zum Rückfluss erwärmt. Es entsteht eine klare braune Lösung. Nach 3 h ist die Reaktion beendet. Die Lösung wird warm filtriert und im Vakuum eingedampft. Es hinterbleibt ein gelbes Oel, das ohne Reinigung weiterverwendet wird.

b) Umsetzung des Carbonsäurechlorides

In 50 ml Toluol werden 0,065 Mol Diisooctylamin und 0,15 Mol Triethylamin gelöst. Die Lösung wird unter Argon auf 0-5 °C abgekühlt. Bei dieser Temperatur lässt man unter Rühren eine Lösung von 0,064 Mol des unter a) beschriebenen Säurechlorides in 50 ml Toluol zutropfen. Hierbei fällt das Triethylamin-Hydrochlorid aus. Nach dem Zutropfen wird die Suspension noch 2 h gerührt und filtriert. Das Filtrat wird mit verdünnter Salzsäure und Wasser gewaschen, über $Na_2SO_4$ getrocknet und eingedampft. Der dunkelgelbe ölige Rückstand wird über einer Kieselgel-Säule chromatographisch gereinigt. Man erhält das 2-[2-Hydroxy-3-ethyl-5-(1,1-dimethyl-4-(diisooctylaminocarbonyl)-butyl)-phenyl]-benztriazol (Verbindung Nr. 33) als gelbliches Harz.
Analyse: N ber. 9,48 % gef. 9,38 %
In analoger Weise werden die in Tabelle 3 aufgeführten Verbindungen erhalten.

Tabelle 3

| Verbindung Nr. | Formel | Physikal. Eigenschaften Analyse |
|---|---|---|
| 34 | 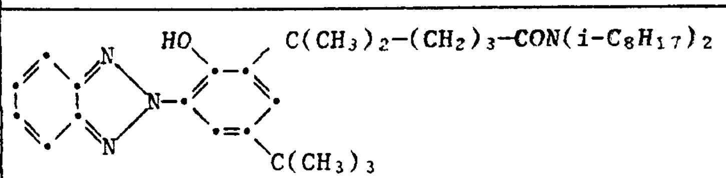 | Harz<br>N = 8,99 %<br>ber. 9,05 % |
| 35 | 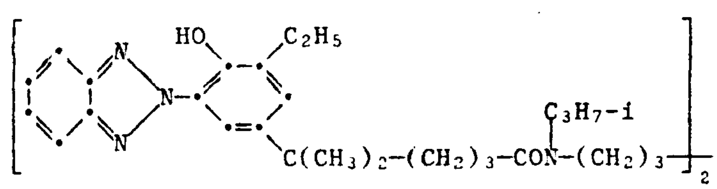 | Harz<br>N = 12,44 %<br>ber. 12,46 % |
| 36 | 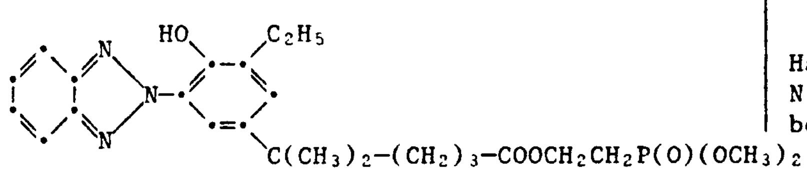 | Harz<br>N = 8,57 %<br>ber. 8,34 % |

Beispiel 5: Stabilisierung einer Magenta-Schicht

Auf ein mit Polyethylen beschichtetes Trägermaterial wird zuerst eine Gelatineschicht aufgetragen, die Silberbromid und einen Magenta-Kuppler enthält, und dann eine Gelatineschicht, die den erfindungsgemässen UV-Absorber enthält (Deckschicht).

Die Gelatineschichten bestehen aus folgenden Komponenten (je $m^2$ Trägermaterial):

| Komponente | AgBr-Schicht | Deckschicht |
|---|---|---|
| Gelatine | 5,15 g | 1,3 g |
| Härtungsmittel | 300 mg | 80 mg |
| Netzmittel | 85 mg | 100 mg |
| Silberbromid | 520 mg | - |
| Trikresylphosphat | 164 mg | 510 mg |
| Magentakuppler | 329 mg | - |
| UV-Absorber | - | 1,1 mMol |

Der Magentakuppler hat die folgende Konstitution:

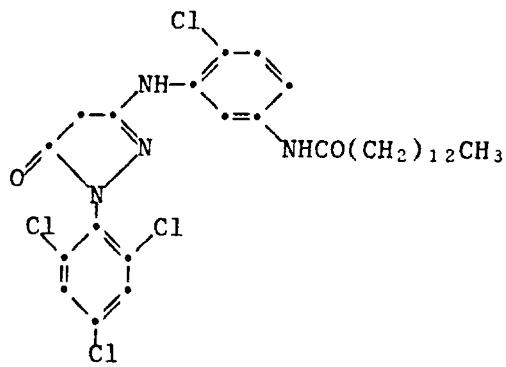

Als Härtungsmittel wird 2,4-Dichlor-6-hydroxytriazin verwendet. Als Netzmittel wird das Natriumsalz von Diisobutylnaphthalinsulfonsäure verwendet.

Auf die so erhaltenen Proben wird jeweils ein Stufenkeil mit einem Dichteunterschied von 0,15 logE pro Stufe aufbelichtet und anschliessend gemäss den Vorschriften des Herstellers im Verarbeitungsprozess E+2 der Firma Kodak für Negativ-Farbpapiere verarbeitet.

Anschliessend wird die Remissionsdichte im Blau für die Vergilbung gemessen. Dann wird der Keil in einem Atlas-Belichtungsgerät mit total 15 kJ/$cm^2$ belichtet, erneut die Remissionsdichte (im Blau) gemessen und die Gelbfarbstoffzunahme berechnet. Die Resultate mit verschiedenen UV-Absorbern sind in Tabelle 4 aufgeführt.

Tabelle 4

| UV-Absorber | Gelbzunahme ($\Delta D^b_{min}$) |
|---|---|
| Keiner | 0,35 |
| Verbindung Nr. 2 | 0,17 |
| Verbindung Nr. 18 | 0,17 |
| Verbindung Nr. 19 | 0,16 |

Beispiel 6: Stabilisierung einer Gelbschicht

Es wird wie in Beispiel 5 gearbeitet unter Verwendung des Gelbkupplers A der folgenden Formel:

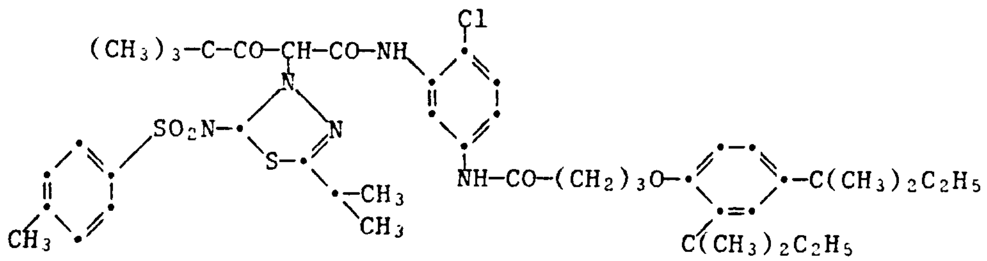

Die Zusammensetzung der Gelatineschichten (pro $m^2$) ist folgende:

| | AgBr-Schicht | Deckschicht |
|---|---|---|
| Gelatine | 5,15 g | 1,3 g |
| Härtungsmittel | 300 mg | 80 mg |
| Netzmittel | 340 mg | 100 mg |
| Silberbromid | 520 mg | - |
| Trikresylphosphat | 309 mg | 510 mg |
| Gelbkuppler A | 927 mg | - |
| UV-Absorber | - | 1,1 mMol |

Nach Belichtung und Verarbeitung wie in Beispiel 5 beschrieben, wird die Remissionsdichte im Blau für die Gelbstufe mit einer Dichte zwischen 0,9 und 1,1 des Keils gemessen. Dann wird der Keil in einem Atlas-Belichtungsgerät mit total 15 kJ/$cm^2$ belichtet und erneut die Remissionsdichte gemessen.

Aufgrund der so erhaltenen Werte lassen sich die in der Tabelle 5 aufgeführten prozentualen Dichteverluste berechnen.

Tabelle 5

| UV-Absorber | Dichteverlust |
|---|---|
| Keiner | 33 % |
| Verbindung Nr. 2 | 16 % |
| Verbindung Nr. 18 | 17 % |
| Verbindung Nr. 19 | 15 % |

Beispiel 7: Stabilisierung einer Cyanschicht

Es wird wie in Beispiel 5 gearbeitet, jedoch unter Verwendung des Cyankupplers der folgenden Formel:

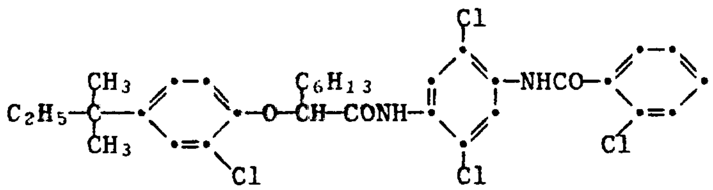

Die Zusammensetzung der Gelatineschichten ist folgende (pro $m^2$):

| | AgBr-Schicht | Deckschicht |
|---|---|---|
| Gelatine | 5,15 g | 1,3 g |
| Härtungsmittel | 300 mg | 80 mg |
| Netzmittel | 170 mg | 100 mg |
| Silberbromid | 260 mg | - |
| Trikresylphosphat | 220 mg | 510 mg |
| Cyankuppler | 331 mg | - |
| UV-Absorber | - | 1,1 mMol |

Nach Belichtung und Verarbeitung wie in Beispiel 5 beschrieben, wird die Remissionsdichte im Rot für die Cyanstufe mit einer Dichte zwischen 0,9 und 1,1 des Keils gemessen. Dann wird der Keil in einem Atlas-Belichtungsgerät mit total 15 $kJ/cm^2$ belichtet und erneut die Remissionsdichte gemessen.

Aufgrund der so erhaltenen Werte lassen sich die in der Tabelle 6 aufgeführten prozentualen Dichteverluste berechnen.

Tabelle 6

| UV-Absorber | Dichteverlust |
|---|---|
| Keiner | 21 % |
| Verbindung Nr. 2 | 15 % |
| Verbindung Nr. 18 | 15 % |
| Verbindung Nr. 19 | 14 % |

Beispiel 8: Stabilisierung einer Gelbschicht

Es wird wie in Beispiel 5 gearbeitet, jedoch unter Verwendung des Gelbkupplers B der folgenden Formel:

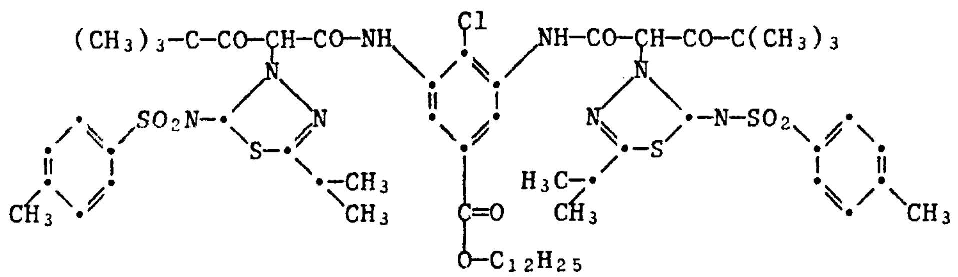

Die Zusammensetzung der Gelatineschichten (pro $m^2$) ist folgende:

| | AgBr-Schicht | Deckschicht |
|---|---|---|
| Gelatine | 5,15 g | 1,3 g |
| Härtungsmittel | 300 mg | 80 mg |
| Netzmittel (anionisch) | 340 mg | 100 mg |
| Silberbromid | 520 mg | - |
| Trikresylphosphat | 214 mg | 510 mg |
| Gelbkuppler $\beta$ | 641 mg | - |
| UV-Absorber | - | 1,1 mMol |

Nach Belichtung und Verarbeitung wie in Beispiel 5 beschrieben, wird die Remissionsdichte im Blau für die Gelbstufe mit einer Dichte zwischen 0,9 und 1,1 des Keils gemessen. Dann wird der Keil in einem Atlas-Belichtungsgerät mit total 15 $kJ/cm^2$ belichtet und erneut die Remissionsdichte gemessen.

Aufgrund der so erhaltenen Werte lassen sich die in der Tabelle 7 aufgeführten prozentualen Dichteverluste berechnen.

Tabelle 7

| UV-Absorber | Dichteverlust |
|---|---|
| Keiner | 59 % |
| Verbindung Nr. 2 | 31 % |
| Verbindung Nr. 18 | 33 % |
| Verbindung Nr. 19 | 31 % |

**Patentansprüche**

**1.** Verbindung der Formel I

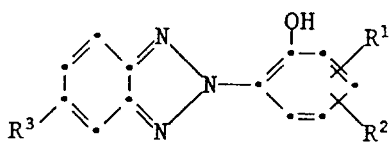

I

worin $R^1$ in ortho- oder para-Stellung zur OH-Gruppe steht und eine Gruppe der Formel II bedeutet,

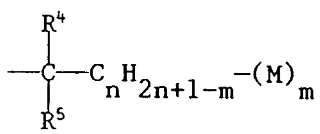

II

worin $R^4$ und $R^5$ gleich oder verschieden sind und $C_1$-$C_5$-Alkyl bedeuten oder $R^4$ zusammen mit dem Rest $C_nH_{2n+1-m}$ einen $C_5$-$C_{12}$-Cycloalkylenring bildet,
m 1 oder 2 bedeutet,
n eine ganze Zahl von 2 bis 20 ist und
M einer der folgenden Reste ist:
a) $-COOR^6$ oder

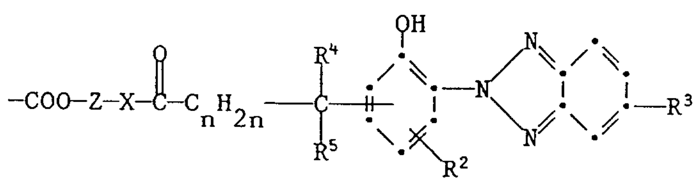

worin $R^6$ Wasserstoff, unsubstituiertes oder durch -OH, $-O-CO-R^7$ oder $-P(O)(OR^8)_2$ substituiertes $C_1$-$C_{18}$-Alkyl, durch ein oder mehrere -O-, -S-oder $-N(R^9)-$ unterbrochenes $C_3$-$C_{30}$-Alkyl oder -Hydroxyalkyl, unsubstituiertes oder durch -OH substituiertes $C_5$-$C_{12}$-Cycloalkyl, $C_2$-$C_{18}$-Alkenyl, $C_7$-$C_{15}$-Aralkyl, Glycidyl, Furfuryl, eine Gruppe $-CH_2-CH(OH)-R^{10}$, einen einwertigen Mono- oder Disaccharid-Rest oder eine Gruppe der Formel III

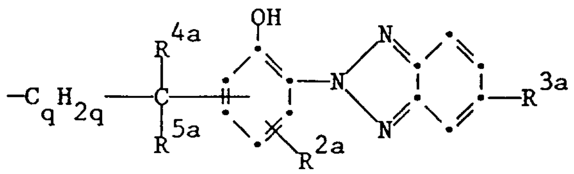

(III)

bedeutet, worin q eine ganze Zahl von 2 bis 20 ist und $R^{4a}$ und $R^{5a}$ $C_1$-$C_5$-Alkyl bedeuten,
X -O- oder -N($R^9$)- bedeutet,
Z $C_2$-$C_8$-Alkylen, $C_4$-$C_8$-Alkenylen, $C_4$-$C_8$-Alkinylen, Cyclohexylen, durch ein oder mehrere -O- unterbrochenes $C_4$-$C_{40}$-Alkylen oder $-CH_2CH(OH)CH_2-OR^{11}O-CH_2CH(OH)CH_2-$ bedeutet,
$R^7$ $C_1$-$C_{18}$-Alkyl, $C_5$-$C_{12}$-Cycloalkyl, $C_2$-$C_8$-Alkenyl, $C_6$-$C_{10}$-Aryl, $C_7$-$C_{18}$-Alkylaryl oder $C_7$-$C_{15}$-Aralkyl bedeutet,
$R^8$ $C_1$-$C_{18}$-Alkyl, $C_5$-$C_{12}$-Cycloalkyl, $C_3$-$C_8$-Alkenyl, $C_6$-$C_{10}$-Aryl oder $C_7$-$C_{15}$-Aralkyl bedeutet,
$R^9$ Wasserstoff, $C_1$-$C_{18}$-Alkyl, $C_5$-$C_{12}$-Cycloalkyl, $C_3$-$C_8$-Alkenyl, $C_6$-$C_{10}$-Aryl, $C_7$-$C_{18}$-Alkylaryl oder $C_7$-$C_{15}$-Aralkyl bedeutet,
$R^{10}$ Wasserstoff, $C_1$-$C_{12}$-Alkyl, Phenyl, Hydroxyphenyl, $C_7$-$C_{15}$-Aralkyl oder $-CH_2OR^7$ bedeutet und
$R^{11}$ $C_2$-$C_8$-Alkylen, durch -O- unterbrochenes $C_4$-$C_{10}$-Alkylen, Cyclohexylen, Phenylen oder eine Gruppe

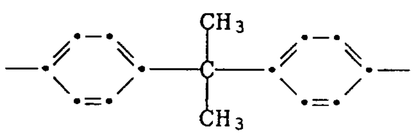

oder

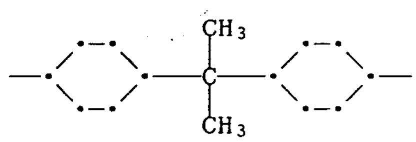

bedeutet,
b) -CO-N($R^{12}$)($R^{13}$) oder

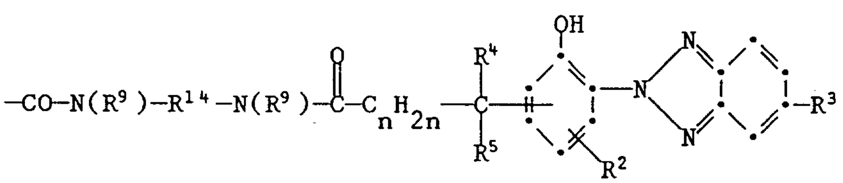

,

worin $R^{12}$ und $R^{13}$ unabhängig voneinander H, $C_1$-$C_{18}$-Alkyl, durch ein oder mehrere -O-, -S- oder -N($R^9$)- unterbrochenes $C_3$-$C_{30}$-Alkyl, $C_5$-$C_{12}$-Cycloalkyl, Phenyl oder durch $C_1$-$C_{12}$-Alkyl, $C_1$-$C_4$-Alkoxy oder Halogen substituiertes Phenyl, $C_3$-$C_6$-Alkenyl, $C_7$-$C_{15}$-Aralkyl, $C_2$-$C_4$-Hydroxyalkyl oder eine Gruppe der Formel III bedeuten oder
$R^{12}$ und $R^{13}$ zusammen $C_4$-$C_6$-Alkylen oder durch -O-, -S- oder -N($R^9$)-unter-brochenes $C_4$-$C_6$-Alkylen bedeuten und
$R^{14}$ $C_2$-$C_{12}$-Alkylen, durch -O- unterbrochenes $C_4$-$C_{12}$-Alkylen, Cyclohexylen, Phenylen oder eine Gruppe

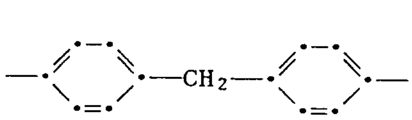

oder

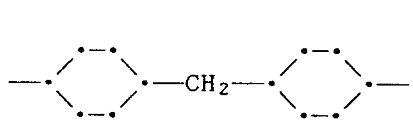

bedeutet oder zusammen mit den beiden N-Atomen und den beiden Resten $R^9$ einen Piperazinring bildet,

c) $-OR^{12}$ oder

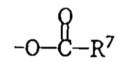

wobei $R_7$ verschieden von Vinyl und Isopropenyl ist, wenn $R^2$ Wasserstoff, Methyl oder tert. Alkyl mit 4 bis 6 Kohlenstoffatomen ist, und $R^{12}$ verschieden von Wasserstoff ist, wenn $R^2$ in ortho-Stellung zur OH-Gruppe steht und Wasserstoff, Methyl oder Alkyl mit 4 bis 6 Kohlenstoffatomen ist.

d) $-N(R^{16})(R^{17})$,

worin $R^{16}$ Wasserstoff oder $C_1$-$C_{12}$-Alkyl ist und

$R^{17}$ Wasserstoff, $C_1$-$C_{12}$-Alkyl oder $-CO-R^9$ ist oder

$R^{16}$ und $R^{17}$ zusammen $C_4$-$C_6$-Alkylen oder durch -O-, -S- oder $-N(R^9)$-unterbrochenes $C_4$-$C_6$-Alkylen bedeuten,

e) $-P(O)(OR^{18})(OR^{19})$ oder $-P(O)(OR^{18})-R^{20}$,

worin $R^{18}$ Wasserstoff oder $C_1$-$C_{18}$-Alkyl ist,

$R^{19}$ $C_1$-$C_{18}$-Alkyl ist, oder $R^{18}$ und $R^{19}$ zusammen $C_2$-$C_5$-Alkylen bedeuten und

$R^{20}$ $C_1$-$C_{12}$-Alkyl oder Phenyl bedeutet,

f) -CN, $-NO_2$ oder Halogen,

g)

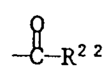 ,

worin $R^{22}$ Wasserstoff, $C_1$-$C_{20}$-Alkyl oder Halogen bedeutet;

$R^2$ und $R^{2a}$ in ortho- oder para-Stellung zur OH-Gruppe steht und Wasserstoff, Halogen, $C_1$-$C_{18}$-Alkyl, $C_7$-$C_9$-Phenylalkyl, $C_5$-$C_8$-Cycloalkyl, Phenyl, eine Gruppe der Formel II oder eine Gruppe der Formel IV, V oder VI bedeutet,

$-C_pH_{2p}-COOR^6$ IV

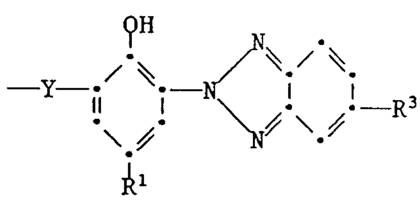

V

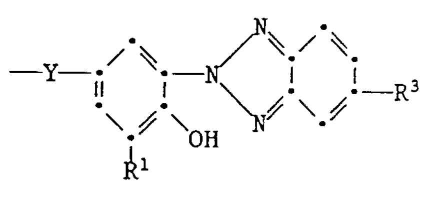

VI

worin p 0,1 oder 2 ist und Y eine direkte Bindung, -S- oder eine Gruppe

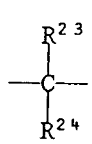

ist, worin $R^{23}$ und $R^{24}$ unabhängig voneinander Wasserstoff, $C_1$-$C_{18}$-Alkyl oder durch 1 bis 3 -S- unterbrochenes $C_3$-$C_{20}$-Alkyl bedeuten und

$R^3$ und $R^{3a}$ Wasserstoff, Chlor, $C_1$-$C_4$-Alkyl, $C_1$-$C_4$-Alkoxy oder -$COOR^6$ bedeutet.

**2.** Verbindung gemäss Anspruch 1 der Formel I, worin $R^1$ eine Gruppe der Formel IIa ist,

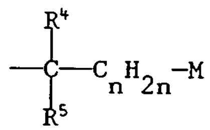

IIa

worin $R^4$ und $R^5$ unabhängig voneinander $C_1$-$C_5$-Alkyl sind oder $R^4$ zusammen mit dem Rest $C_nH_{2n}$ einen $C_5$-$C_6$-Cycloalkylenring bildet,
n eine ganze Zahl von 2 bis 8 ist,
M eine der in Anspruch 1 gegebenen Bedeutungen hat,
$R^2$ $C_1$-$C_{12}$-Alkyl oder eine Gruppe der Formel IIa oder eine Gruppe der Formel IV bedeutet, worin p 1 oder 2 ist, und
$R^3$ Wasserstoff, Chlor, $C_1$-$C_4$-Alkyl, $C_1$-$C_4$-Alkoxy oder -$COOR^6$ bedeutet, worin $R^6$ die in Anspruch 1 angegebenen Bedeutungen hat.

**3.** Verbindung gemäss Anspruch 1, worin M eine Gruppe -$COOR^6$ oder

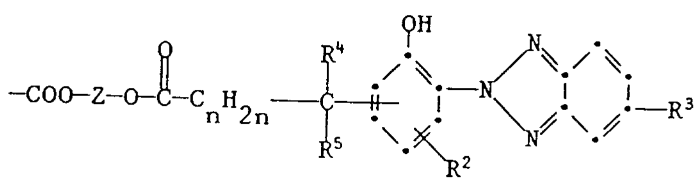

ist,

worin $R^6$ Wasserstoff, unsubstituiertes oder durch -OH substituiertes $C_1$-$C_{18}$-Alkyl, durch ein oder mehrere -O- unterbrochenes $C_3$-$C_{20}$-Alkyl oder Hydroxyalkyl, unsubstituiertes oder durch -OH substituiertes $C_5$-$C_{12}$-Cycloalkyl, $C_2$-$C_{18}$-Alkenyl, $C_7$-$C_{15}$-Aralkyl, Glycidyl, Furfuryl oder eine Gruppe -$CH_2$-CH-(OH)-$R^{10}$ bedeutet und $R^{10}$ Wasserstoff, $C_1$-$C_{12}$-Alkyl, $C_7$-$C_{12}$-Aralkyl oder -$CH_2OR^7$ bedeutet und $R^7$ $C_1$-$C_{18}$-Alkyl, Cyclohexyl, Phenyl Tolyl oder Benzyl ist,
Z $C_2$-$C_8$-Alkylen, $C_4$-$C_8$-Alkenylen, Cyclohexylen, durch ein oder mehrere -O- unterbrochenes $C_4$-$C_{40}$-Alkylen oder -$CH_2CH(OH)CH_2$-$OR^{11}$-O-$CH_2CH(OH)CH_2$-bedeutet,
$R^{11}$ $C_2$-$C_8$-Alkylen oder durch ein oder mehrere -O- unterbrochenes $C_4$-$C_{10}$-Alkylen bedeutet
und $R^2$, $R^3$, $R^4$, $R^5$ und n die in Anspruch 1 gegebenen Bedeutungen haben.

**4.** Verbindung der Formel I

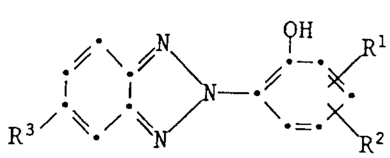

I

worin $R^1$ in ortho- oder para-Stellung zur OH-Gruppe steht und eine Gruppe der Formel II bedeutet,

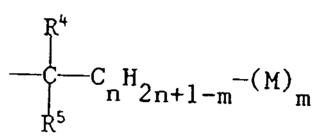

II

worin $R^4$ und $R^5$ gleich oder verschieden sind und $C_1$-$C_5$-Alkyl bedeuten oder $R^4$ zusammen mit dem Rest $C_nH_{2n+1-m}$ einen $C_5$-$C_{12}$-Cycloalkylenring bildet,
m 1 oder 2 bedeutet,

n eine ganze Zahl von 2 bis 20 ist
$R^2$ und $R^{2a}$ in ortho- oder para-Stellung zur OH-Gruppe steht und Wasserstoff, Halogen, $C_1$-$C_{18}$-Alkyl, $C_7$-$C_9$-Phenylalkyl, $C_5$-$C_8$-Cycloalkyl, Phenyl, eine Gruppe der Formel II oder eine Gruppe der Formel IV, V oder VI bedeutet,

$-C_pH_{2p}-COOR^6$ IV

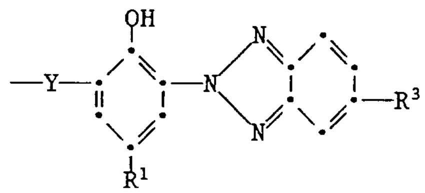

V

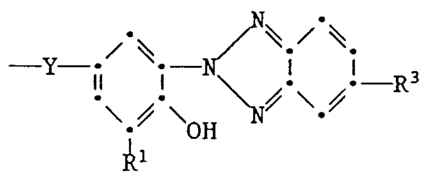

VI

worin p 0,1 oder 2 ist und Y eine direkte Bindung, -S- oder eine Gruppe

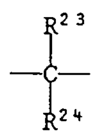

ist, worin $R^{23}$ und $R^{24}$ unabhängig voneinander Wasserstoff,
$C_1$-$C_{18}$-Alkyl oder durch 1 bis 3 -S- unterbrochenes $C_3$-$C_{20}$-Alkyl bedeuten und
$R^3$ und $R^{3a}$ Wasserstoff, Chlor, $C_1$-$C_4$-Alkyl, $C_1$-$C_4$-Alkoxy oder $-COOR^6$ bedeutet,
$R^6$ Wasserstoff, unsubstituiertes oder durch -OH, $-O-CO-R^7$ oder $-P(O)(OR^8)_2$ substituiertes $C_1$-$C_{18}$-Alkyl, durch ein oder mehrere -O-, -S-oder $-N(R^9)$- unterbrochenes $C_3$-$C_{30}$-Alkyl oder -Hydroxyalkyl, unsubstituiertes oder durch -OH substituiertes $C_5$-$C_{12}$-Cycloalkyl, $C_2$-$C_{18}$-Alkenyl, $C_7$-$C_{15}$-Aralkyl, Glycidyl, Furfuryl, eine Gruppe $-CH_2-CH(OH)-R^{10}$, einen einwertigen Mono- oder Disaccharid-Rest oder eine Gruppe der Formel III

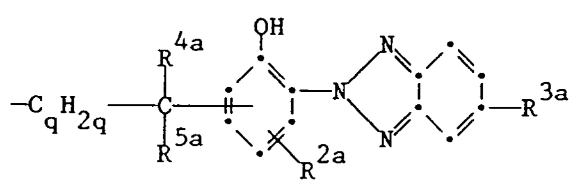

(III)

bedeutet, worin q eine ganze Zahl von 2 bis 20 ist und $R^{4a}$ und $R^{5a}$ $C_1$-$C_5$-Alkyl bedeuten,
$R^7$ $C_1$-$C_{18}$-Alkyl, $C_5$-$C_{12}$-Cycloalkyl, $C_2$-$C_8$-Alkenyl, $C_6$-$C_{10}$-Aryl, $C_7$-$C_{18}$-Alkylaryl oder $C_7$-$C_{15}$-Aralkyl bedeutet,
$R^8$ $C_1$-$C_{18}$-Alkyl, $C_5$-$C_{12}$-Cycloalkyl, $C_3$-$C_8$-Alkenyl, $C_6$-$C_{10}$-Aryl oder $C_7$-$C_{15}$-Aralkyl bedeutet,
$R^9$ Wasserstoff, $C_1$-$C_{18}$-Alkyl, $C_5$-$C_{12}$-Cycloalkyl, $C_3$-$C_8$-Alkenyl, $C_6$-$C_{10}$-Aryl, $C_7$-$C_{18}$-Alkylaryl oder $C_7$-$C_{15}$-Aralkyl bedeutet,
$R^{10}$ Wasserstoff, $C_1$-$C_{12}$-Alkyl, Phenyl, Hydroxyphenyl, $C_7$-$C_{15}$-Aralkyl oder $-CH_2OR^7$ bedeutet und
M eine Gruppe $-CO-N(R^{12})(R^{13})$ oder

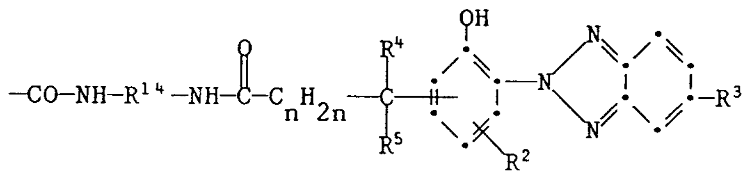 ist,

worin $R^{12}$ und $R^{13}$ unabhängig voneinander H, $C_1$-$C_{12}$-Alkyl, durch -O-unterbrochenes $C_3$-$C_{20}$-Alkyl, $C_5$-$C_{12}$-Cycloalkyl, Phenyl oder durch $C_1$-$C_{12}$-Alkyl, $C_1$-$C_4$-Alkoxy oder Halogen substituiertes Phenyl, $C_3$-$C_8$-Alkenyl, $C_7$-$C_{11}$-Phenylalkyl, oder $C_2$-$C_4$-Hydroxyalkyl bedeuten oder $R^{12}$ und $R^{13}$ zusammen $C_4$-$C_5$-Alkylen oder durch -O- oder -N($R^9$)- unterbrochenes $C_4$-$C_6$-Alkylen bedeuten und
$R^{14}$ $C_2$-$C_8$-Alkylen oder durch -O- unterbrochenes $C_4$-$C_8$-Alkylen bedeutet.

**5.** Verbindung gemäss Anspruch 1, worin M eine Gruppe -$OR^{12}$ oder

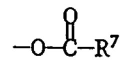

ist, worin $R^{12}$ Wasserstoff, $C_1$-$C_{12}$-Alkyl, $C_5$-$C_{12}$-Cycloalkyl, $C_3$-$C_6$-Alkenyl, Phenyl oder $C_7$-$C_{11}$-Phenylalkyl bedeutet und $R_7$ $C_1$-$C_{18}$-Alkyl, Cyclohexyl, $C_2$-$C_6$-Alkenyl, Phenyl oder $C_7$-$C_{12}$-Alkylphenyl bedeutet.

**6.** Verbindung gemäss Anspruch 1, worin M eine Gruppe -P(O)($OR^{18}$)($OR^{19}$) oder -P(O)($OR^{18}$)-$R^{20}$ ist, worin $R^{18}$ Wasserstoff oder $C_1$-$C_{12}$-Alkyl, $R^{19}$ $C_1$-$C_{12}$-Alkyl ist, und $R^{20}$ $C_1$-$C_{12}$-Alkyl oder Phenyl bedeutet.

**7.** Verbindung gemäss Anspruch 1, worin M eine Gruppe

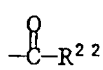

ist, worin $R^{22}$ $C_1$-$C_{12}$-Alkyl bedeutet.

**8.** Verbindung gemäss Anspruch 1 der Formel I,
worin $R^1$ eine Gruppe der Formel IIa ist,
worin $R^4$ und $R^5$ Methyl sind und n eine ganze Zahl von 2 bis 6 ist,
M eine Gruppe -$COOR^6$ oder -OH ist, worin
$R^6$ Wasserstoff, unsubstituiertes oder durch -OH substituiertes $C_1$-$C_{18}$-Alkyl, durch ein oder mehrere -O- unterbrochenes $C_3$-$C_{30}$-Alkyl oder -Hydroxyalkyl, unsubstituiertes oder durch -OH substituiertes $C_5$-$C_{12}$-Cycloalkyl, $C_2$-$C_{18}$-Alkenyl, $C_7$-$C_{15}$-Aralkyl, Glycidyl oder Furfuryl bedeutet,
$R^2$ $C_1$-$C_{12}$-Alkyl, $C_7$-$C_9$-Phenylalkyl, $C_5$-$C_8$-Cycloalkyl, eine Gruppe der Formel II oder der Formel IV bedeutet, worin p 1 oder 2 ist und
$R^3$ Wasserstoff, Chlor, $C_1$-$C_4$-Alkyl, $C_1$-$C_4$-Alkoxy oder -$COOR^6$ bedeutet.

**9.** Verbindung gemäss Anspruch 1 der Formel I, worin $R^1$ eine Gruppe der Formel IIb ist,

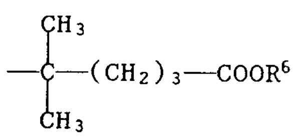 IIb

worin $R^6$ unsubstituiertes oder durch -OH substituiertes $C_5$-$C_{18}$-Alkyl, durch ein oder mehrere -O-unterbrochenes $C_3$-$C_{30}$-Alkyl oder -Hydroxyalkyl, $C_5$-$C_{12}$-Cycloalkyl, $C_2$-$C_{18}$-Alkenyl, $C_7$-$C_{15}$-Aralkyl, Glycidyl oder Furfuryl bedeutet,
$R^2$ $C_1$-$C_{12}$-Alkyl, Cyclohexyl oder eine Gruppe der Formel IIb oder IV bedeutet, worin p 1 oder 2 ist,

und
$R^3$ Wasserstoff, Chlor, $C_1$-$C_4$-Alkyl oder $C_1$-$C_4$-Alkoxy bedeutet.

**10.** Verbindung gemäss Anspruch 1 der Formel Ia,

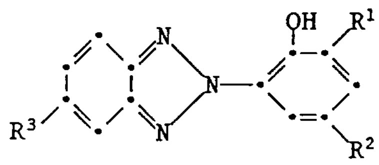

Ia

worin $R^1$ eine Gruppe der Formel IIb ist, worin $R^6$ $C_5$-$C_{12}$-Alkyl, durch ein oder mehrere -O-unterbrochenes $C_3$-$C_{18}$-Alkyl, $C_5$-$C_{12}$-Cycloalkyl, $C_3$-$C_{18}$-Alkenyl oder $C_7$-$C_{12}$-Phenylalkyl bedeutet,
$R^2$ dieselbe Bedeutung wie $R^1$ hat oder $C_1$-$C_{12}$-Alkyl oder eine Gruppe der Formel IV ist, worin p 1 oder 2 bedeutet, und
$R^3$ Wasserstoff, Chlor oder $C_1$-$C_4$-Alkyl bedeutet.

**11.** Verwendung einer Verbindung des Anspruches 1 als Stabilisator für organische Materialien.

**12.** Verwendung gemäss Anspruch 11 als Stabilisator für Lacke.

**13.** Verwendung gemäss Anspruch 11 als Stabilisator für photographische Materialien.

**14.** Stabilisiertes organisches Material, enthaltend mindestens eine Verbindung des Anspruches 1.

**15.** Organisches Material gemäss Anspruch 14, enthaltend 0,01 bis 5 Gew.-%, bezogen auf das Material, mindestens einer Verbindung des Anspruches 1.

**16.** Stabilisiertes photographisches Material gemäss Anspruch 14.

**17.** Stabilisierter Lack gemäss Anspruch 14.

## Claims

**1.** A compound of the formula I

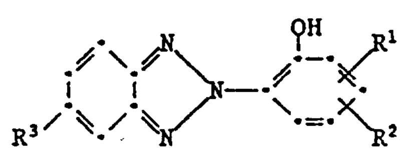

I

in which $R^1$ is located in the ortho-position or para-position relative to the OH group and is a group of the formula II

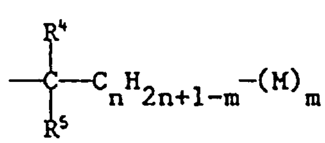

II

in which $R^4$ and $R^5$ are identical or different and are $C_1$-$C_5$alkyl, or $R^4$, together with the radical $C_nH_{2n+1-m}$, forms a $C_5$-$C_{12}$cycloalkylene ring, m is 1 or 2, n is an integer from 2 to 20 and M is one of the following radicals:

a) $-COOR^6$ or

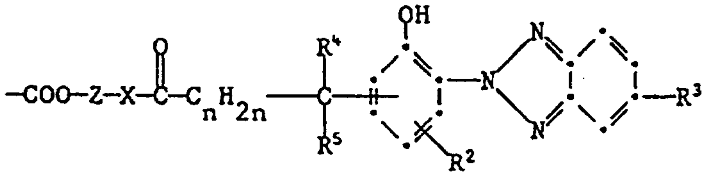

in which $R^6$ is hydrogen, $C_1$-$C_{18}$alkyl which is unsubstituted or substituted by -OH, $-O-CO-R^7$ or $-P-(O)(OR^8)_2$, $C_3$-$C_{30}$alkyl or $C_3$-$C_{30}$hydroxyalkyl each of which is interrupted by one or more -O-, -S- or $-N(R^9)-$ groups, $C_5$-$C_{12}$cycloalkyl, $C_2$-$C_{18}$alkenyl, $C_7$-$C_{15}$aralkyl, glycidyl or furfuryl each of which is unsubstituted or substituted by -OH, a group $-CH_2-CH(OH)-R^{10}$, a monovalent monosaccharide or disaccharide radical or a group of the formula III

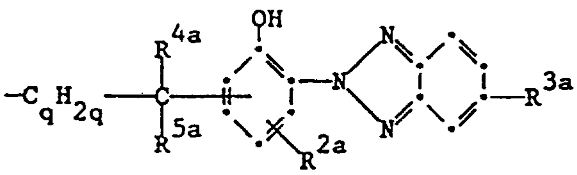

(III)

in which q is an integer from 2 to 20 and $R^{4a}$ and $R^{5a}$ are $C_1$-$C_5$alkyl, X is -O-or $-N(R^9)-$, Z is $C_2$-$C_8$alkylene, $C_4$-$C_8$alkenylene, $C_4$-$C_8$alkynylene, cyclohexylene, $C_4$-$C_{40}$alkylene which is interrupted by one or more -O- groups or is $-CH_2CH(OH)CH_2-OR^{11}O-CH_2CH(OH)CH_2-$, $R^7$ is $C_1$-$C_{18}$alkyl, $C_5$-$C_{12}$cycloalkyl, $C_2$-$C_8$alkenyl, $C_6$-$C_{10}$aryl, $C_7$-$C_{18}$alkylaryl or $C_7$-$C_{15}$aralkyl, $R^8$ is $C_1$-$C_{18}$alkyl, $C_5$-$C_{12}$cycloalkyl, $C_3$-$C_8$alkenyl, $C_6$-$C_{10}$arylor $C_7$-$C_{15}$aralkyl, $R^9$ is hydrogen, $C_1$-$C_{18}$alkyl, $C_5$-$C_{12}$cycloalkyl, $C_3$-$C_8$alkenyl, $C_6$-$C_{10}$aryl, $C_7$-$C_{18}$alkylaryl or $C_7$-$C_{15}$aralkyl, $R^{10}$ is hydrogen, $C_1$-$C_{12}$alkyl, phenyl, hydroxyphenyl, $C_7$-$C_{15}$aralkyl or $-CH_2OR^7$ and $R^{11}$ is $C_2$-$C_8$alkylene, $C_4$-$C_{10}$alkylene which is interrupted by -O-, cyclohexylene, phenylene or a group

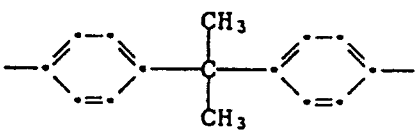

or

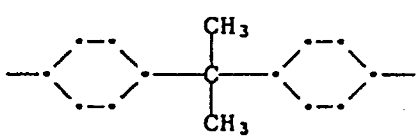

b) $-CO-N(R^{12})(R^{13})$ or

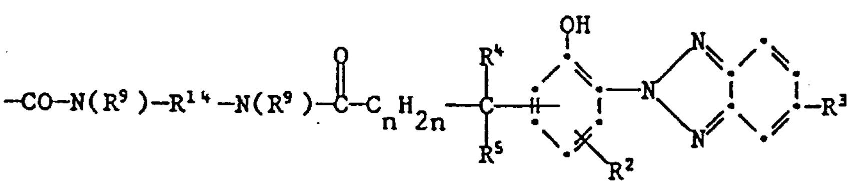

,

in which $R^{12}$ and $R^{13}$ independently of one another are H, $C_1$-$C_{18}$alkyl, $C_3$-$C_{30}$alkyl which is interrupted by one or more -O-, -S- or $-N(R^9)-$ groups, $C_5$-$C_{12}$cycloalkyl, phenyl or phenyl which is substituted by $C_1$-$C_{12}$alkyl, $C_1$-$C_4$alkoxy or halogen, $C_3$-$C_6$alkenyl, $C_7$-$C_{15}$aralkyl, $C_2$-$C_4$hydroxyalkyl or a group of the formula III or $R^{12}$ and $R^{13}$ together are $C_4$-$C_6$alkylene or $C_4$-$C_6$alkylene which is interrupted by -O-, -S- or $-N(R^9)-$ and $R^{14}$ is $C_2$-$C_{12}$alkylene, $C_4$-$C_{12}$alkylene which is interrupted by -O-, cyclohexylene, phenylene or a group

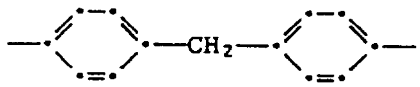 or 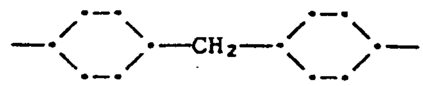

or together with the two N atoms and the two radicals $R^9$ forms a piperazine ring,
c) $-OR^{12}$ or

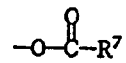

where $R^7$ is different from vinyl and isopropenyl, if $R^2$ is hydrogen, methyl or tert-alkyl having 4 to 6 carbon atoms, and $R^{12}$ is different from hydrogen, if $R^2$ is in the ortho-position relative to the OH group and is hydrogen, methyl or alkyl having 4 to 6 carbon atoms,
d) $-N(R^{16})(R^{17})$ in which $R^{16}$ is hydrogen or $C_1$-$C_{12}$alkyl and $R^{17}$ is hydrogen, $C_1$-$C_{12}$alkyl or $-CO-R^9$ or $R^{16}$ and $R^{17}$ together are $C_4$-$C_6$alkylene or $C_4$-$C_6$alkylene which is interrupted by -O-, -S- or $-N(R^9)-$,
e) $-P(O)(OR^{18})(OR^{19})$ or $-P(O)(OR^{18})-R^{20}$ in which $R^{18}$ is hydrogen or $C_1$-$C_{18}$alkyl, $R^{19}$ is $C_1$-$C_{18}$alkyl or $R^{18}$ and $R^{19}$ together are $C_2$-$C_5$alkylene and $R^{20}$ is $C_1$-$C_{12}$alkyl or phenyl,
f) -CN, $-NO_2$ or halogen, or
g)

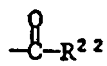 ,

in which $R^{22}$ is hydrogen, $C_1$-$C_{20}$alkyl or halogen; $R^2$ and $R^{2a}$ are located in the ortho-position or para-position relative to the OH group and are hydrogen, halogen, $C_1$-$C_{18}$alkyl, $C_7$-$C_9$phenylalkyl, $C_5$-$C_8$cycloalkyl, phenyl, a group of the formula II or a group of the formula IV, V or VI

$-C_pH_{2p}-COOR^6$ IV

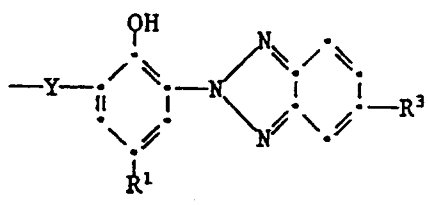 V

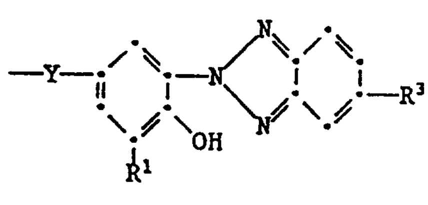 VI

in which p is 0, 1 or 2 and Y is a direct bond, -S- or a group in which $R^{23}$ and $R^{24}$ independently of one another are hydrogen,

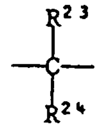

$C_1$-$C_{18}$alkyl or $C_3$-$C_{20}$alkyl which is interrupted by 1 to 3 -S-groups, and $R^3$ and $R^{3a}$ are hydrogen, chlorine, $C_1$-$C_4$alkyl, $C_1$-$C_4$alkoxy or -$COOR^6$.

**2.** A compound according to claim 1 of the formula I in which $R^1$ is a group of the formula IIa

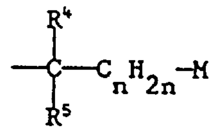

IIa

in which $R^4$ and $R^5$ independently of one another are $C_1$-$C_5$alkyl, or $R^4$, together with the radical $C_nH_{2n}$, forms a $C_5$-$C_6$cycloalkylene ring, n is an integer from 2 to 8, M has one of the meanings given in claim 1, $R^2$ is $C_1$-$C_{12}$alkyl or a group of the formula IIa or a group of the formula IV in which p is 1 or 2, and $R^3$ is hydrogen, chlorine, $C_1$-$C_4$alkyl, $C_1$-$C_4$alkoxy or -$COOR^6$ in which $R^6$ is as defined in claim 1.

**3.** A compound according to claim 1, in which M is a group -$COOR^6$ or

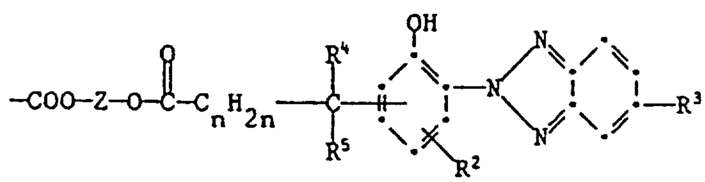

in which $R^6$ is hydrogen, $C_1$-$C_{18}$alkyl which is unsubstituted or substituted by -OH, $C_3$-$C_{20}$alkyl or $C_3$-$C_{20}$hydroxyalkyl each of which is interrupted by one or more -O- groups, $C_5$-$C_{12}$cycloalkyl, $C_2$-$C_{18}$alkenyl, $C_7$-$C_{15}$aralkyl, glycidyl or furfuryl each of which is unsubstituted or substituted by -OH, or is a group -$CH_2$-CH(OH)-$R^{10}$ and $R^{10}$ is hydrogen, $C_1$-$C_{12}$alkyl, $C_7$-$C_{12}$aralkyl or -$CH_2OR^7$ and $R^7$ is $C_1$-$C_{18}$alkyl, cyclohexyl, phenyl, tolyl or benzyl, Z is $C_2$-$C_8$alkylene, $C_4$-$C_8$alkenylene, cyclohexylene, $C_4$-$C_{40}$alkylene which is interrupted by one or more -O- groups, or is -$CH_2CH(OH)CH_2$-$OR^{11}$-O-$CH_2CH$-(OH)$CH_2$-, $R^{11}$ is $C_2$-$C_8$alkylene or $C_4$-$C_{10}$alkylene which is interrupted by one or more -O- groups, and $R^2$, $R^3$, $R^4$, $R^5$ and n are as defined in claim 1.

**4.** A compound of the formula I

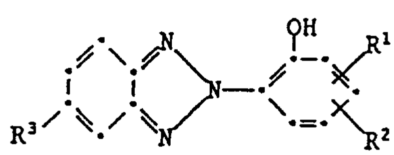

I

in which $R^1$ is located in the ortho-position or para-position relative to the OH group and is a group of the formula II

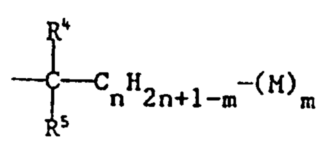

II

in which $R^4$ and $R^5$ are identical or different and are $C_1$-$C_5$alkyl, or $R^4$, together with the radical $C_nH_{2n+1-m}$, forms a $C_5$-$C_{12}$cycloalkylene ring, m is 1 or 2, n is an integer from 2 to 20 and $R^2$ and $R^{2a}$ are located in the ortho-position or para-position relative to the OH group and are hydrogen, halogen, $C_1$-$C_{18}$alkyl, $C_7$-$C_9$phenylalkyl, $C_5$-$C_8$cycloalkyl, phenyl, a group of the formula II or a group of the

formula IV, V or VI

$-C_pH_{2p}-COOR^6$ IV

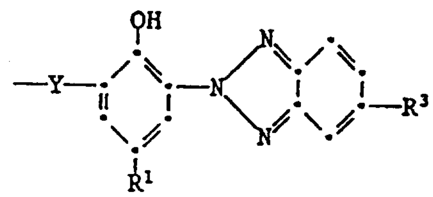

V

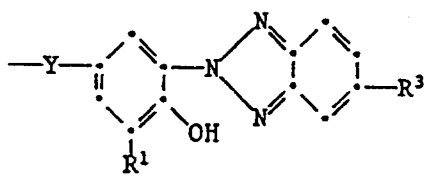

VI

in which p is 0, 1 or 2 and Y is a direct bond, -S- or a group

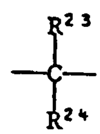

In which $R^{23}$ and $R^{24}$ independently of one another are hydrogen, $C_1$-$C_{18}$alkyl or $C_3$-$C_{20}$alkyl which is interrupted by 1 to 3 -S-groups, and $R^3$ and $R^{3a}$ are hydrogen, chlorine, $C_1$-$C_4$alkyl, $C_1$-$C_4$alkoxy or $-COOR^6$,

$R^6$ is hydrogen, $C_1$-$C_{18}$alkyl which is unsubstituted or substituted by -OH, $-O-CO-R^7$ or $-P(O)(OR^8)_2$, $C_3$-$C_{30}$alkyl or $C_3$-$C_{30}$hydroxyalkyl each of which is interrupted by one or more -O-, -S- or $-N(R^9)$-groups, $C_5$-$C_{12}$cycloalkyl, $C_2$-$C_{18}$alkenyl, $C_7$-$C_{15}$aralkyl, glycidyl or furfuryl each of which is unsubstituted or substituted by -OH, a group $-CH_2-CH(OH)-R^{10}$, a monovalent monosaccharide or disaccharide radical or a group of the formula III

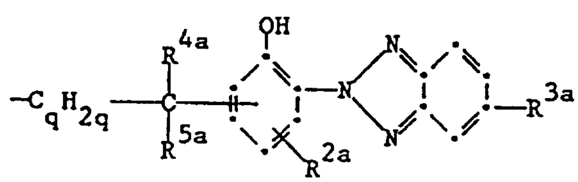

(III)

in which q is an integer from 2 to 20 and $R^{4a}$ and $R^{5a}$ are $C_1$-$C_5$alkyl, $R^7$ is $C_1$-$C_{18}$alkyl, $C_5$-$C_{12}$cycloalkyl, $C_2$-$C_8$alkenyl, $C_6$-$C_{10}$aryl, $C_7$-$C_{18}$alkylarylor $C_7$-$C_{15}$aralkyl, $R^8$ is $C_1$-$C_{18}$alkyl, $C_5$-$C_{12}$cycloalkyl, $C_3$-$C_8$alkenyl,

$C_6$-$C_{10}$aryl or $C_7$-$C_{15}$aralkyl, $R^9$ is hydrogen, $C_1$-$C_{18}$alkyl, $C_5$-$C_{12}$cycloalkyl, $C_3$-$C_8$alkenyl, $C_6$-$C_{10}$aryl, $C_7$-$C_{18}$alkylaryl or $C_7$-$C_{15}$aralkyl, $R^{10}$ is hydrogen, $C_1$-$C_{12}$alkyl, phenyl, hydroxyphenyl, $C_7$-$C_{15}$aralkyl or $-CH_2OR^7$ and M is a group $-CO-N(R^{12})(R^{13})$ or

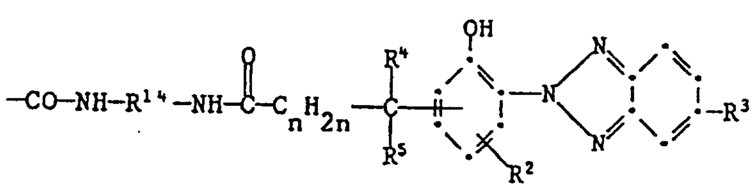

in which $R^{12}$ and $R^{13}$ independently of one another are H, $C_1$-$C_{12}$alkyl, $C_3$-$C_{20}$alkyl which is interrupted by -O-, $C_5$-$C_{12}$cycloalkyl, phenyl or phenyl which is substituted by $C_1$-$C_{12}$alkyl, $C_1$-$C_4$alkoxy or halogen, $C_3$-$C_8$alkenyl, $C_7$-$C_{11}$phenylalkyl or $C_2$-$C_4$hydroxyalkyl, or $R^{12}$ and $R^{13}$ together are $C_4$-$C_5$alkylene or $C_4$-$C_6$alkylene which is interrupted by -O-or -N($R^9$)-, and $R^{14}$ is $C_2$-$C_8$alkylene or $C_4$-$C_8$alkylene which is interrupted by -O-.

**5.** A compound according to claim 1, in which M is a group -$OR^{12}$ or

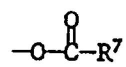

in which $R^{12}$ is hydrogen, $C_1$-$C_{12}$alkyl, $C_5$-$C_{12}$cycloalkyl, $C_3$-$C_6$alkenyl, phenyl or $C_7$-$C_{11}$phenylalkyl and $R_7$ is $C_1$-$C_{18}$alkyl, cyclohexyl, $C_2$-$C_6$alkenyl, phenyl or $C_7$-$C_{12}$alkylphenyl.

**6.** A compound according to claim 1, in which M is a group -P(O)($OR^{18}$)($OR^{19}$) or -P(O)($OR^{18}$)-$R^{20}$ in which $R^{18}$ is hydrogen or $C_1$-$C_{12}$alkyl, $R^{19}$ is $C_1$-$C_{12}$alkyl and $R^{20}$ is $C_1$-$C_{12}$alkyl or phenyl.

**7.** A compound according to claim 1, in which M is a group

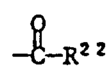

in which $R^{22}$ is $C_1$-$C_{12}$alkyl.

**8.** A compound according to claim 1 of the formula I in which $R^1$ is a group of the formula IIa in which $R^4$ and $R^5$ are methyl and n is an integer from 2 to 6, M is a group -$COOR^6$ or -OH in which $R^6$ is hydrogen, $C_1$-$C_{18}$alkyl which is unsubstituted or substituted by -OH, $C_3$-$C_{30}$alkyl or $C_3$-$C_{30}$hydroxyalkyl each of which is interrupted by one or more -O-groups, or $C_5$-$C_{12}$cycloalkyl, $C_2$-$C_{18}$alkenyl, $C_7$-$C_{15}$aralkyl, glycidyl or furfuryl each of which is unsubstituted or substituted by -OH, $R^2$ is $C_1$-$C_{12}$alkyl, $C_7$-$C_9$phenylalkyl, $C_5$-$C_8$cycloalkyl or a group of the formula II or of the formula IV in which p is 1 or 2 and $R^3$ is hydrogen, chlorine, $C_1$-$C_4$alkyl, $C_1$-$C_4$alkoxy or -$COOR^6$.

**9.** A compound according to claim 1 of the formula I in which $R^1$ is a group of the formula IIb

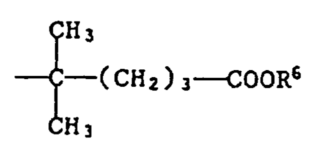

IIb

in which $R^6$ is $C_5$-$C_{18}$alkyl which is unsubstituted or substituted by -OH, $C_3$-$C_{30}$alkyl or $C_3$-$C_{30}$hydroxyalkyl each of which is interrupted by one or more -O- groups, or is $C_5$-$C_{12}$cycloalkyl, $C_2$-$C_{18}$alkenyl, $C_7$-$C_{15}$aralkyl, glycidyl or furfuryl, $R^2$ is $C_1$-$C_{12}$alkyl, cyclohexyl or a group of the formula IIb or IV in which p is 1 or 2, and $R^3$ is hydrogen, chlorine, $C_1$-$C_4$alkyl or $C_1$-$C_4$alkoxy.

**10.** A compound according to claim 1 of the formula Ia

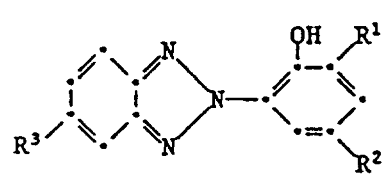

Ia

in which $R^1$ is a group of the formula IIb in which $R^6$ is $C_5$-$C_{12}$alkyl, $C_3$-$C_{18}$alkyl which is interrupted by

one or more -O- groups, $C_5$-$C_{12}$cycloalkyl, $C_3$-$C_{18}$alkenyl or $C_7$-$C_{12}$phenylalkyl, $R^2$ is as defined for $R^1$ or is $C_1$-$C_{12}$alkyl or a group of the formula IV in which p is 1 or 2, and $R^3$ is hydrogen, chlorine or $C_1$-$C_4$alkyl.

**11.** The use of a compound of claim 1 as a stabilizer for organic materials.

**12.** Use according to claim 11 as a stabilizer for coating materials.

**13.** Use according to claim 11 as a stabilizer for photographic materials.

**14.** Stabilized organic material containing at least one compound of claim 1.

**15.** Organic material according to claim 14, containing 0.01 to 5% by weight, relative to the material, of at least one compound of claim 1.

**16.** Stabilized photographic material according to claim 14.

**17.** Stabilized coating material according to claim 14.

## Revendications

**1.** Composé de formule I :

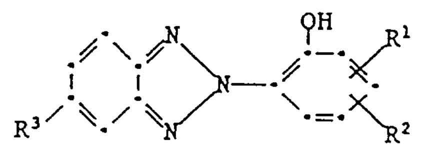

(I)

dans laquelle $R^1$ est en position ortho ou para par rapport au groupe OH et signifie un groupe de formule II :

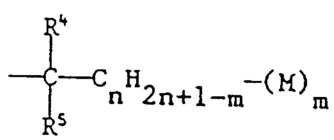

(II)

dans laquelle $R^4$ et $R^5$ sont identiques ou différents et signifient un alkyle en $C_1$-$C_5$ ou $R^4$ ensemble avec le radical $C_nH_{2n+1-m}$ forme un cycle de cycloalkylène en $C_5$-$C_{12}$,
m signifie 1 ou 2,
n est un nombre entier de 2 à 20, et
M est un des radicaux suivants :
a) -$COOR^6$ ou

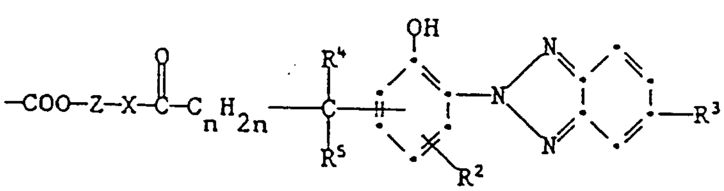

où $R^6$ signifie l'hydrogène, un alkyle en $C_1$-$C_{18}$ non substitué ou substitué par -OH, -O-CO-$R^7$ ou -P-(O) $(OR^8)_2$, un hydroxyalkyle ou alkyle en $C_3$-$C_{30}$ interrompu par un ou plusieurs -O-, -S- ou -N($R^9$)-, un aralkyle en $C_7$-$C_{15}$, un alcényle en $C_2$-$C_{18}$, un cycloalkyle en $C_5$-$C_{12}$ non substitué ou substitué

par -OH, un glycidyle, un furfuryle, un groupe $-CH_2-CH(OH)-R^{10}$, un radical mono-ou disaccharide monovalent ou un groupe de formule III :

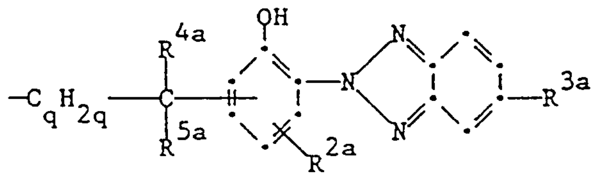

(III)

dans laquelle q est un nombre entier de 2 à 20 et $R^{4a}$ et $R^{5a}$ signifient un alkyle en $C_1$-$C_5$,
X signifie -O- ou -N ($R^9$) -,
Z signifie un alkylène en $C_2$-$C_8$, un alcénylène en $C_4$-$C_8$, un alcynylène en $C_4$-$C_8$, un cyclohexylène, $-CH_2-CH(OH)CH_2-OR^{11}O-CH_2CH(OH)CH_2-$ ou un alkylène en $C_4$-$C_{40}$ interrompu par un ou plusieurs -O-,
$R^7$ signifie un alkyle en $C_1$-$C_{18}$, un cycloalkyle en $C_5$-$C_{12}$, un alcényle en $C_2$-$C_8$, un aryle en $C_6$-$C_{10}$, un alkylaryle en $C_7$-$C_{18}$ ou un aralkyle en $C_7$-$C_{15}$,
$R^8$ signifie un alkyle en $C_1$-$C_{18}$, un cycloalkyle en $C_5$-$C_{12}$, un alcényle en $C_3$-$C_8$, un aryle en $c_6$-$c_{10}$ ou un aralkyle en $C_7$-$C_{15}$,
$R^9$ signifie l'hydrogène, un alkyle en $C_1$-$C_{18}$, un cycloalkyle en $C_5$-$C_{12}$, un alcényle en $C_3$-$C_8$, un aryle en $C_6$-$C_{10}$, un alkylaryle en $C_7$-$C_{18}$ ou un aralkyle en $C_7$-$C_{15}$,
$R^{10}$ signifie l'hydrogène, un alkyle en $C_1$-$C_{12}$, un phényle, un hydroxyphényle, un aralkyle en $C_7$-$C_{15}$ ou $-CH_2OR^7$, et
$R^{11}$ signifie un alkylène en $C_2$-$C_8$, un phénylène, un cyclohexylène, un alkylène en $C_4$-$C_{10}$ interrompu par -O-ou un groupe :

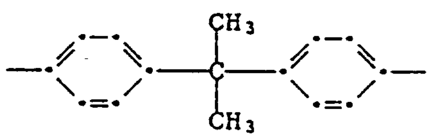

ou

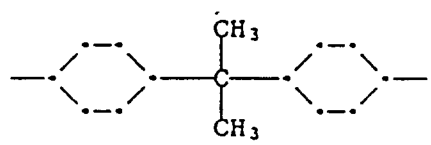

b) $-CO-N(R^{12})(R^{13})$ ou

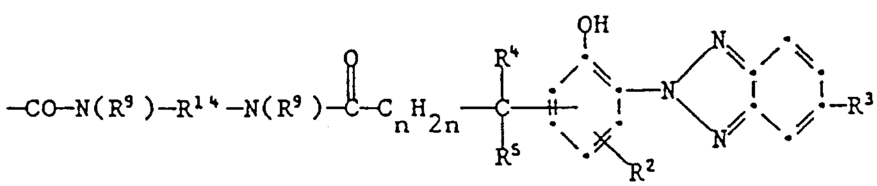

où $R^{12}$ et $R^{13}$ indépendamment l'un de l'autre signifient H, un alkyle en $C_1$-$C_{18}$, un cycloalkyle en $C_5$-$C_{12}$, un alkyle en $C_3$-$C_{30}$ interrompu par un ou plusieurs -O-, -S-ou $-N(R^9)-$, un phényle ou un phényl substitué par un alkyle en $C_1$-$C_{12}$, par un alcoxy en $C_1$-$C_4$ ou par un halogène, un alcényle en $C_3$-$C_6$, un aralkyle en $C_7$-$C_{15}$, un hydroxyalkyle en $C_2$-$C_4$ ou un groupe de formule III, ou
$R^{12}$ et $R^{13}$ ensemble signifient un alkylène en $C_4$-$C_6$ ou un alkylène en $C_4$-$C_6$ interrompu par -O-, -S- ou -N ($R^9$) - et
$R^{14}$ signifie un alkylène en $C_2$-$C_{12}$, un phénylène, un cyclohexylène, un alkylène en $C_4$-$C_{12}$ interrompu par -O-ou un groupe :

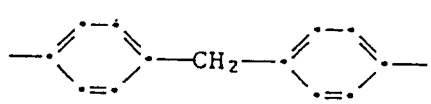

ou

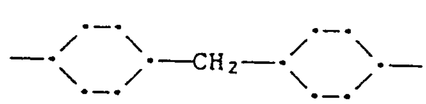

ou ensemble avec les deux atomes d'azote et les deux radicaux $R^9$ forme un cycle de pipérazine,
c) $-OR^{12}$ ou

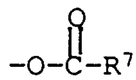

où $R^7$ est différent du vinyle et de l'isopropényle, lorsque $R^2$ est l'hydrogène, un méthyle ou un tert-alkyle avec un nombre d'atomes de carbone de 4 à 6 et $R^{12}$ est différent de l'hydrogène lorsque $R^2$ est en position ortho par rapport au groupe -OH, et est l'hydrogène, un méthyle ou un alkyle avec un nombre d'atomes de carbone de 4 à 6,
d) $-N(R^{16})(R^{17})$,
où $R^{16}$ est l'hydrogène ou un alkyle en $C_1$-$C_{12}$ et
$R^{17}$ est l'hydrogène, un alkyle en $C_1$-$C_{12}$ ou $-CO-R^9$ ou $R^{16}$ et $R^{17}$ ensemble signifient un alkylène en $C_4$-$C_6$ ou un alkylène en $C_4$-$C_6$ interrompu par -O-, -S- ou $-N(R^9)-$,
e) $-P(O)(OR^{18})(OR^{19})$ ou $-P(O)(OR^{18})-R^{20}$,
où $R^{18}$ est l'hydrogène ou un alkyle en $C_1$-$C_{18}$, $R^{19}$ est un alkyle en $C_1$-$C_{18}$ ou $R^{18}$ et $R^{19}$ ensemble signifient un alkylène en $C_2$-$C_5$ et
$R^{20}$ signifie un alkyle en $C_1$-$C_{12}$ ou un phényle,
f) -CN, $-NO_2$ ou halogène, O
g) $-C-R^{22}$,
où $R^{22}$ signifie l'hydrogène, un alkyle en $C_1$-$C_{20}$ ou un halogène ;
$R^2$ et $R^{2a}$ sont en position ortho ou para par rapport au groupe OH et signifient l'hydrogène, un halogène, un alkyle en $C_1$-$C_{18}$, un phénylalkyle en $C_7$-$C_9$, un cycloalkyle en $C_5$-$C_8$, un phényle, un groupe de formule II ou un groupe de formule IV, V ou VI

$-C_pH_{2p}-COOR^6$ IV

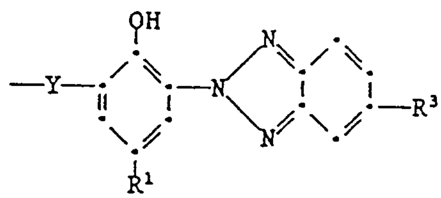
V

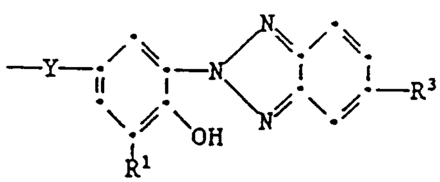
VI

dans lesquelles p est 0,1 ou 2 et Y est une liaison directe, -S- ou un groupe

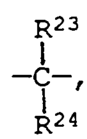

dans lequel $R^{23}$ et $R^{24}$ indépendamment l'un de l'autre signifient l'hydrogène, un alkyle en $C_1$-$C_{18}$ ou un alkyle en $C_3$-$C_{20}$ interrompu par 1 à 3 -S- et
$R^3$ et $R^{3a}$ signifient l'hydrogène, le chlore, un alkyle en $C_1$-$C_4$, un alcoxy en $C_1$-$C_4$ ou $-COOR^6$.

**2.** Composé selon la revendication 1 répondant à la formule I, dans laquelle $R^1$ est un groupe de formule IIa :

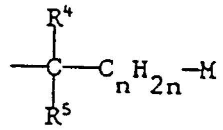

(IIa)

dans laquelle $R^4$ et $R^5$ indépendamment l'un de l'autre sont un alkyle en $C_1$-$C_5$ ou $R^4$ ensemble avec le radical $C_nH_{2n}$ forme un cycle de cycloalkylène en $C_5$-$C_6$,
n est un nombre entier de 2 à 8,
M a l'une des significations données dans la revendication 1,
$R^2$ signifie un alkyle en $C_1$-$C_{12}$ ou un groupe de formule IIa ou un groupe de formule IV dans laquelle p est 1 ou 2, et
$R^3$ signifie l'hydrogène, le chlore, un alkyle en $C_1$-$C_4$,un alcoxy en $C_1$-$C_4$ ou -COOR$^6$, où $R^6$ a les significations données dans la revendication 1.

**3.** Composé selon la revendication 1, où M est un groupe -COOR$^6$ ou

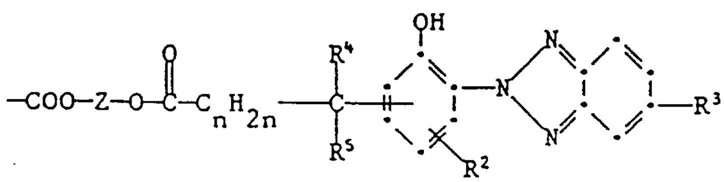

où $R^6$ signifie l'hydrogène, un alkyle en $C_1$-$C_{18}$ non substitué ou substituè par -OH, un hydroxyalkyle ou alkyle en $C_3$-$C_{20}$ interrompu par un ou plusieurs -O-, un aralkyle en $C_7$-$C_{15}$, un alcényle en $C_2$-$C_{18}$, un cycloalkyle en $C_5$-$C_{12}$ non substitué ou substitué par -OH, un glycidyle, un furfuryle ou un groupe $-CH_2-CH(OH)-R^{10}$, et $R^{10}$ signifie l'hydrogène, un alkyle en $C_1$-$C_{12}$, un aralkyle en $C_7$-$C_{12}$ ou $-CH_2OR^7$ et $R^7$ est un alkyle en $C_1$-$C_{18}$, un cyclohexyle, un phényle, un tolyle ou un benzyle,
Z signifie un alkylène en $C_2$-$C_8$, un alcénylène en $C_4$-$C_8$, un cyclohexylène, $-CH_2-CH(OH)CH_2-OR^{11}-O-CH_2CH(OH)CH_2-$ ou un alkylène en $C_4$-$C_{40}$ interrompu par un ou plusieurs -O-ou
$R^{11}$ signifie un alkylène en $C_2$-$C_8$ ou un alkylène en $C_4$-$C_{10}$ interrompu par un ou plusieurs -O-, et
$R^2$, $R^3$, $R^4$, $R^5$ et n ont les significations données dans la revendication 1.

**4.** Composé de formule I :

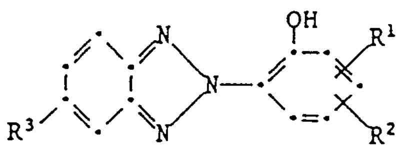

(I)

dans laquelle $R^1$ est en position ortho ou para par rapport au groupe OH et signifie un groupe de formule II :

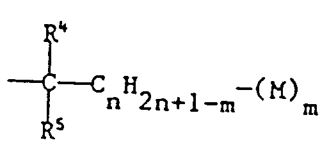

(II)

dans laquelle $R^4$ et $R^5$ sont identiques ou différents et signifient un alkyle en $C_1$-$C_5$ ou $R^4$ ensemble avec le radical $C_nH_{2n+1-m}$ forme un cycle de cycloalkylène en $C_5$-$C_{12}$,
m signifie 1 ou 2,
n est un nombre entier de 2 à 20,
$R^2$ et $R^{2a}$ sont en position ortho ou para par rapport au groupe OH et signifient l'hydrogène, un halogène, un alkyle en $C_1$-$C_{18}$, un phénylalkyle en $C_7$-$C_9$, un cycloalkyle en $C_5$-$C_8$, un phényle, un groupe de formule II ou un groupe de formule IV, V ou VI

$-C_pH_{2p}-COOR^6$ IV

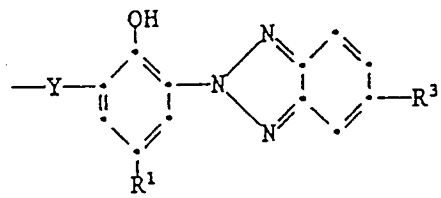

V

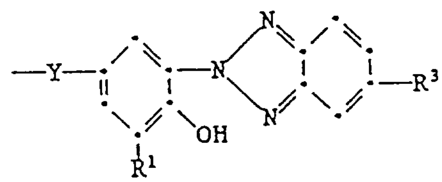

VI

dans lesquelles p est 0,1 ou 2 et Y est une liaison directe, -S- ou un groupe

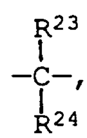

dans lequel $R^{23}$ et $R^{24}$ indépendamment l'un de l'autre signifient l'hydrogène, un alkyle en $C_1$-$C_{18}$ ou un alkyle en $C_3$-$C_{20}$ interrompu par 1 à 3 -S- et
$R^3$ et $R^{3a}$ signifient l'hydrogène, le chlore, un alkyle en $C_1$-$C_4$, un alcoxy en $C_1$-$C_4$ ou $-COOR^6$,
$R^6$ signifie l'hydrogène, un alkyle en $C_1$-$C_{18}$ non substitué ou substitué par -OH, $-O-CO-R^7$ ou -P (O) $(OR^8)_2$, un hydroxyalkyle ou alkyle en $C_3$-$C_{30}$ interrompu par un ou plusieurs -O-, -S- ou -N ($R^9$), un aralkyle en $C_7$-$C_{15}$, un alcényle en $C_2$-$C_{18}$, un cycloalkyle en $C_5$-$C_{12}$ non substitué ou substitué par -OH, un glycidyle, un furfuryle ou un groupe $-CH_2$-CH (OH) $-R^{10}$, un radical monofonctionnel mono- ou disaccharide ou un groupe de formule III :

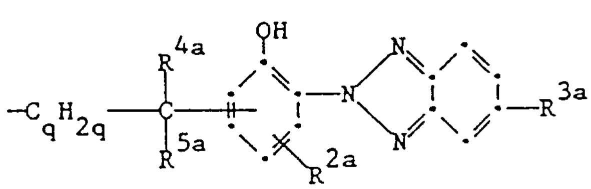

(III)

où q est un nombre entier de 2 à 20 et $R^{4a}$ et $R^{5a}$ signifient un alkyle en $C_1$-$C_5$,
$R^7$ signifie un alkyle en $C_1$-$C_{18}$, un cycloalkyle en $C_5$-$C_{12}$, un alcényle en $C_2$-$C_8$, un aryle en $C_6$-$C_{10}$, un alkylaryle en $C_1$-$C_{18}$ ou un aralkyle en $C_7$-$C_{15}$,
$R^8$ signifie un alkyle en $C_1$-$C_{18}$, un cycloalkyle en $C_5$-$C_{12}$, un alcényle en $C_3$-$C_8$, un aryle en $C_6$-$C_{10}$ ou un aralkyle en $C_7$-$C_{15}$,
$R^9$ signifie l'hydrogène, un alkyle en $C_1$-$c_{18}$, un cycloalkyle en $C_5$-$C_{12}$, un alcényle en $C_3$-$C_8$, un aryle en $C_6$-$C_{10}$, un alkylaryle en $C_7$-$C_{18}$ ou un aralkyle en $C_7$-$C_{15}$,
$R^{10}$ signifie l'hydrogène, un alkyle en $C_1$-$C_{12}$, un phényle, un hydroxyphényle, un aralkyle en $C_7$-$C_{15}$

ou $-CH_2OR^7$,
M est un groupe $-CO-N(R^{12})\ (R^{13})$ ou

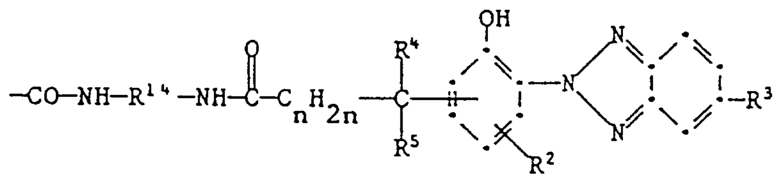

où $R^{12}$ et $R^{13}$ indépendamment l'un de l'autre signifient H, un alkyle en $C_1$-$C_{12}$, un cycloalkyle en $C_5$-$C_{12}$, un alkyle en $C_3$-$C_{20}$ interrompu par -O-, un phényle ou un phényle substitué par un alkyle en $C_1$-$C_{12}$, par un alcoxy en $C_1$-$C_4$ ou par un halogène, un alcényle en $C_3$-$C_8$, un phénylalkyl en $C_7$-$C_{11}$ ou un hydroxyalkyle en $C_2$-$C_4$ ou $R^{12}$ et $R^{13}$ ensemble signifient un alkylène en $C_4$-$C_5$ ou un alkylène en $C_4$-$C_6$ interrompu par -O- ou $-N\ (R^9)$ - et
$R^{14}$ signifie un alkylène en $C_2$-$C_8$ ou un alkylène en $C_4$-$C_8$ interrompu par -O-.

**5.** Composé selon la revendication 1, où M est un groupe $-OR^{12}$ ou

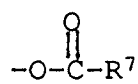

où $R^{12}$ signifie l'hydrogène, un alkyle en $C_1$-$C_{12}$, un cycloalkyle en $C_5$-$C_{12}$, un alcényle en $C_3$-$C_6$, un phényle ou un phénylalkyle en $C_7$-$C_{11}$, et $R^7$ signifie un alkyle en $C_1$-$C_{18}$, un cyclohexyle, un alcényle en $C_2$-$C_6$, un phényle ou un alkylphényle en $C_7$-$C_{12}$.

**6.** Composé selon la revendication 1, où M est un groupe $-P(O)\ (OR^{18})\ (OR^{19})$ ou $-P(O)\ (OR^{18})-R^{20}$, où $R^{18}$ est l'hydrogène ou un alkyle en $C_1$-$C_{12}$, $R^{19}$ est un alkyle en $C_1$-$C_{12}$, et $R^{20}$ signifie un alkyle en $C_1$-$C_{12}$ ou un phényle.

**7.** Composé selon la revendication 1, où M est un groupe

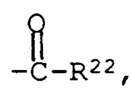

dans laquelle $R^{22}$ signifie un alkyle en $C_1$-$C_{12}$.

**8.** Composé selon la revendication 1 répondant à la formule I,
où $R^1$ est un groupe de formule IIa,
où $R^4$ et $R^5$ sont un méthyle et n est un nombre entier de 2 à 6,
M est un groupe $-COOR^6$ ou -OH, où
$R^6$ signifie l'hydrogène, un alkyle en $C_1$-$C_{18}$ non substitué ou substitué par -OH, un hydroxyalkyle ou alkyle en $C_3$-$C_{30}$ interrompu par un ou plusieurs -O-, un aralkyle en $C_7$-$C_{15}$, un alcényle en $C_2$-$C_{18}$, un cycloalkyle en $C_5$-$C_{12}$ non substitué ou substitué par -OH, un glycidyle ou un furfuryle,
$R^2$ signifie un alkyle en $C_1$-$C_{12}$, un phénylalkyle en $C_7$-$C_9$, un cycloalkyle en $C_5$-$C_8$, un groupe de formule II ou de formule IV, où p est 1 ou 2, et
$R^3$ signifie l'hydrogène, le chlore, un alkyle en $C_1$-$C_4$,un alcoxy en $C_1$-$C_4$ ou $-COOR^6$.

**9.** Composé selon la revendication 1, répondant à la formule I, où $R^1$ est un groupe répondant à la formule IIb :

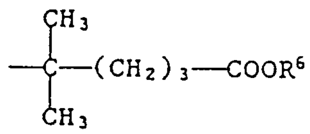

(IIb)

où $R^6$ signifie un alkyle en $C_5$-$C_{18}$ non substitué ou substitué par -OH, un aralkyle en $C_7$-$C_{15}$, un alcényle en $C_2$-$C_{18}$, un cycloalkyle en $C_5$-$C_{12}$, un hydroxyalkyle ou alkyle en $C_3$-$C_{30}$ interrompu par un ou plusieurs -O-, un glycidyle ou un furfuryle,
$R^2$ signifie un alkyle en $C_1$-$C_{12}$, un cyclohexyle ou un groupe de formule IIb ou de formule IV, où p est 1 ou 2, et
$R^3$ signifie l'hydrogène, le chlore, un alkyle en $C_1$-$C_4$ ou un alcoxy en $C_1$-$C_4$.

**10.** Composé selon la revendication 1 répondant à la formule Ia :

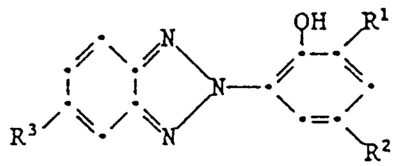

(Ia)

dans laquelle $R^1$ est un groupe de formule IIb,
où $R^6$ est un alkyle en $C_5$-$C_{12}$, un phénylalkyle en $C_7$-$C_{12}$, un alcényle en $C_3$-$C_{18}$, un cycloalkyle en $C_5$-$C_{12}$ ou un alkyle en $C_3$-$C_{18}$ interrompu par un ou plusieurs -O-,
$R^2$ a la même signification que $R^1$ ou est un alkyle en $C_1$-$C_{12}$ ou un groupe de formule IV, où p est 1 ou 2, et
$R^3$ signifie l'hydrogène, le chlore ou un alkyle en $C_1$-$C_4$.

**11.** Utilisation d'un composé de la revendication 1, en tant que stabilisant pour matières organiques.

**12.** Utilisation selon la revendication 11, en tant que stabilisant pour vernis.

**13.** Utilisation selon la revendication 11, en tant que stabilisant pour matériaux photographiques.

**14.** Matières organiques stabilisées, contenant au moins un composé selon la revendication 1.

**15.** Matériaux organiques selon la revendication 14, contenant de 0,01 à 5 % en poids par rapport à la matière, d'au moins un composé de la revendication 1.

**16.** Matières photographiques stabilisés selon la revendication 14.

**17.** Vernis stabilisés selon la revendication 14.